(19) Europäisches Patentamt
European Patent Office
Office européen des brevets

(11) Veröffentlichungsnummer: **0 149 242**
**B1**

(12) **EUROPÄISCHE PATENTSCHRIFT**

(45) Veröffentlichungstag der Patentschrift: **31.10.90**

(51) Int. Cl.[5]: **A 61 K 31/15,** C 07 C 249/04

(21) Anmeldenummer: **84116381.9**

(22) Anmeldetag: **27.12.84**

(54) **1-(2-Hydroxyaryl)-alkan-1-on-oxime, Verfahren zu ihrer Herstellung und ihre Verwendung in Arzneimitteln.**

(30) Priorität: **29.12.83 DD 258874**

(43) Veröffentlichungstag der Anmeldung:
**24.07.85 Patentblatt 85/30**

(45) Bekanntmachung des Hinweises auf die Patenterteilung:
**31.10.90 Patentblatt 90/44**

(84) Benannte Vertragsstaaten:
**AT BE CH DE FR GB IT LI NL SE**

(56) Entgegenhaltungen:
**DE-A-2 305 694 DE-A-2 461 039**

**SYNTHESIS, Band 1, Januar 1982, Seiten 68-69, Georg Thieme Verlag, Stuttgart, DE; S. FUJITA et al.: "The Beckmann rearrangement by means of Phosphoryl chloride/N,N-dimethylacetamide; a novel and conveniet method for preparing benzoxazoles"**

**Die Akte enthält technische Angaben, die nach dem Eingang der Anmeldung eingereicht wurden und die nicht in dieser Patentschrift enthalten sind.**

(73) Patentinhaber: **VEB Fahlberg-List Magdeburg
Alt Salbke 60-63
DDR-3013 Magdeburg (DD)**

(72) Erfinder: **Schewe, Tankred, Dr.
Karl-Maron-Strasse 32
DD-1140 Berlin (DD)**
Erfinder: **Kühn, Hartmut, Dr.
Pettenkoferstrasse 39
DD-1035 Berlin (DD)**
Erfinder: **Rapoport, Samuel Mitja, Prof-Dr.
Kuckhoffstrasse 45
DD-1110 Berlin (DD)**
Erfinder: **Binte, Hans-Joachim, Dr.
Erzweg 3
DD-9200 Freiberg (DD)**
Erfinder: **Beger, Jörg, Prof.-Dr.
Maxim-Gorki-Strasse 39
DD-9200 Freiberg (DD)**
Erfinder: **Slapke, Jürgen, Dr.
Kollwitzstrasse 41
DD-1055 Berlin (DD)**
Erfinder: **Grupe, Renate, Dr.
Leninalle 218
DD-1156 Berlin (DD)**

(74) Vertreter: **Zipse + Habersack
Kemnatenstrasse 49
D-8000 München 19 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 149 242 B1

Courier Press, Leamington Spa, England.

**Beschreibung**

Andwendungsgebiet der Erfindung

Die Erfindung betrifft teilweise neue 1-(2-Hydroxyaryl)alkan-1-on-oxime und ihre Anwendung in pharmazeutischen Zubereitungen. Die Verbindungen als solche und die sie enthaltenden pharmazeutischen Mittel zeigen wertvolle pharmakologische, insbesondere antiasthmatische, antianaphylaktische, antiphlogistische, antihypertensive, spasmolytische, antirheumatische und antithrombotische Eigenschaften und sind in der Human- und Veterinärmedizin für die Anwendung in der Therapie des Asthma bronchiale und anderer allegischer Erkrankungen, von entzündlichen und rheumatischen Erkrankungen verschiedener Art sowie der Thrombose geeignet.

Charakteristik der bekannten technischen Lösungen

Verfahren zur Herstellung von Ketoximen sind an sich hinreichend bekannt und in jedem Handbuch über organische Synthesen (vgl. Weygand-Hilgetag, Organisch-Chemische Experimentierkunst) zu finden. Für die Umsetzung von 1-Aryl-alkan-1-onen mit Hydroxylaminhydrochlorid zu den Oximen sind in der Literatur verschiedene Methoden beschrieben. Allerdings sind dabei häufig die Reaktionsbedingungen wie Reaktionszeiten, Art der verwendeten Base u.a. in Abhängigkeit von der Struktur des Ausgangsketonsnicht optimal gewählt.

Von den 1-Aryl-alkan-1-on-oximen zeichnen sich besonders die 1-(2-Hydroxyphenyl)-alkan-1-on-oxime durch ihre Verwendung in der Metallanalytik als Metallreagentien und in der Hydrometallurgie als selektive Metallextraktionsmittel für die Flüssig-Flüssig-Extraktion aus (DE—OS 23 42 878).

In der DE—OS 23 05 694 werden Verfahren zur Extraktion von Metallwersstoffen aus wäßrigen Lösungen z.B. die Extraktion von Kupfer mit n-Undecyl-2-hydroxy-5-methylphenyl-ketoxim beschrieben.

Das 5-iso-Nonyl-2-hydroxy-acetophenon-oxim hat für die Gewinnung von Kupfer technische Bedeutung erlangt (Extr. Metall Copper Int. Symp. 1976, 1039, DE—OS 24 07 200).

Desweiteren sind Ketoxime zur Herstellung von Chelatkomplexen zur Stabilisierung von Polymeren bekannt (US—PS 3,579,556).

S. Fujita et. al. beschreibt (Synthesis, Band 1, Januar 1982, 68/69) die Verwendung von Oximen zur Herstellung von Benzoxazole durch Beckmann-Umlagerung. Die Verwendung von 2-Hydroxy-5-methyl-acetophenon-oxim in Arzneimittlen ist bekannt (DE—OS 24 61 039).

Aufgabe der Erfindung

Aufgabe der Erfindung ist die Bereitstellung von pharmazeutischen Zubereitungen mit pharmakologisch wertvollen, insbesondere antiasthmatischen, antianaphylaktischen, antiphlogistischen, antirheumatischen, antihypertensiven, spasmolytischen und antithrombotischen Eigenschaften und von neuen 1-(2-Hydroxyaryl)-alkan-1-on-oximen.

Diese Aufgabe wird gelöst durch pharmazeutische Zusammensetzungen nach Anspruch 1, neue 1-(2-Hydroxyaryl)-alkan-1-on-oxime nach Anspruch 2, durch Mittel gemäß den Ansprüchen 4 bis 11 sowie durch die Verwendung dieser Zusammensetzungen und Stoffe gemäß Anspruch 3 und durch ein Verfahren nach Anspruch 12.

Darlegung des Wesens der Erfindung

Überraschenderweise wurde gefunden, daß 1-(2-Hydroxyaryl)-alkan-1-on-oxime der allgemeinen Formel I gemäß Anspruch 1 auf der Hemmung von Lipoxygenase beruhende, pharmakologisch wertvolle, insbesondere antiasthmatische, antiallergische, entzündungshemmende, antirheumatische, antihypertensive, spasmolytische und antithrombotische Eigenschaften besitzen und als wirksame Bestandteile von pharmazeutischen Zubereitungen verwendet werden können.

Die Verbindungen der allgemeinen Formel I erhält man, indem man eine aliphatische Carbonsäure $C_nH_{2n+1}COOH$ oder ein geeignetes Derivat davon, vorzugsweise ein Carbonsäurechlorid, unter Friedel-Crafts- oder Friedel-Crafts-ähnlichen Acylierungsbedingungen mit einem substituierten Phenol umsetzt. Falls das entstandene Keton noch eine weitere Hydroxylgruppe enthält, wird diese mit einem Alkylierungsmittel, z.B. Alkylhalogeniden, Aralkyl- oder Cycloalkylbromid- oder chlorid, vorzugsweise jedoch mit einem Alkylbromid verethert.

Das Keton wird dann in bekannter Weise mit Hydroxylaminhydrochlorid oder einem anderen geeigneten Hydroxylaminsalz in basischem Medium zum Oxim umsetzt. Es können dazu anorganische oder organische Basen verwendet werden, wie z.B. Natrium- oder Kaliumhydroxid, Calciumhydroxid, Natrium- oder Kaliumcarbonat, Natriumhydrogencarbonat, Natrium- oder Kaliumacetat, Natriumformiat, Pyridin, Piperidin, Morpholin, N-Methylpiperazin, Diethanolamin u.a. Die Veretherung und die Oximierung werden in organischen Lösungsmitteln durchgeführt, die Veretherungerfolgt vorzugsweise in Aceton oder niederen alipahtischen Alkoholen unter Zusatz eines vorstehend aufgeführten basischen Stoffes, insbesondere Kaliumcarbonat oder Natriumhydroxid, die Oximbildung in niederen aliphatischen Alkoholen bzw. Glykolen, Acetonitril, Dioxan, jedoch vorzugsweise in Ethanol, oder in deren Gemischen mit Wasser, wobei im Temperaturbereich von 50—130°C gearbeitet wird. Die Reaktionszeiten für die Alkylierungsreaktion liegen zwischen 15 und 30 Stünden und für die Oximierungsreaktion zwischen 15 Minuten und 3 Stunden.

Nach diesem Verfahren konnten teilweise auch neue, in der Literatur bisher nicht beschriebene und hinsichtlich Elementaranalyse, Schmelzpunkt und IR-spektraler Eigenschaften charakterisierte Verbindungen erhalten werden. Die synthetisierten Verbindungen sind in Tabelle 1 zuammengestellt.

TABELLE 1

1-(2-Hydroxylaryl)-alkan-1-on-oxime

| Verbindung | Fp (°C) | $\nu_{max}$ (OH) | (KBr; $cm^{-1}$) (C=N) | (N—O) |
|---|---|---|---|---|
| 4-Methyl-2-hydroxy-caprophenon-oxim | 45 | 3390 | 1630 | 980 |
| 5-Methyl-2-hydroxy-caprophenon-oxim | 83—85 | 3380 | 1628 | 982 |
| 5-Methyl-2-hydroxy-caprophenon-(N-phenylcarbamoyl)-oxim | 117—120 | | | |
| 5-Methyl-2-hydroxy-laurophenon-oxim | 93 | 3380 | 1626 | 972 |
| 3-Chlor-2-hydroxy-caprophenon-oxim | 102—103 | 3390 | 1638 | 975 |
| 4-Pentoxy-2-hydroxy-acetophenon-oxim | 66—67 | 3420 | 1640 | 980 |
| 4-Decyloxy-2-hydroxy-acetophenon-oxim | 69—70 | 3425<br>3350 | 1628s* | 975 |
| 4-Benzyloxy-2-hydroxy-acetophenon-oxim | 143 | 3360 | 1630 | 962 |
| 4-Decyloxy-2-hydroxy-propiophenon-oxim | 58—59 | 3390 | 1632 | 990 |
| 4-Butoxy-5-n-hexyl-2-hydroxy-acetophenon-oxim | 62 | 3410 | 1640s* | 970 |
| 4-Pentoxy-2-hydroxy-caprophenon-oxim | 80 | 3360 | 1640 | 985 |
| 4-Decyloxy-2-hydroxy-caprophenon-oxim | 57 | 3430 | 1648 | 980 |
| 4-Octyloxy-2-hydroxy-laurophenon-oxim | 60—63 | 3430 | 1640s* | 985 |
| 4-Cyclohexyloxy-2-hydroxy-propiophenon-oxim | 65—69 | 3440 | 1640 | 988 |
| 5-Chlor-2-hydroxy-caprophenon-oxim | 100 | 3385<br>3335s* | 1640 | 990 |
| 3-Chlor-2-hydroxy-laurophenon-oxim | 96 | 3395 | 1640 | 972 |
| 5-Chlor-2-hydroxy-laurophenon-oxim | 97 | 3380 | 1635 | 970 |
| 4-Butoxy-2-hydroxy-acetophenon-oxim | 87 | 3422 | 1640 | 988 |

TABELLE 1 (Fortsetzung)

| Verbindung | Fp (°C) | $v_{max}$ (OH) | (KBr; $cm^{-1}$) (C=N) | (N—O) |
|---|---|---|---|---|
| 4-Dodecyloxy-2-hydroxy-propiophenon-oxim | 63 | 3395 | 1635 | 988 |
| 4-Hexadecyloxy-2-hydroxy-acetophenon-oxim | 86—87 | 3425<br>3350 | 1625 | 973 |
| 4-Octadecyloxy-2-hydroxy-acetophenon-oxim | 90 | 3410<br>3360s* | 1627 | 980 |
| 4-Decyloxy-2-hydroxy-laurophenon-oxim | 64—69 | 3430 | 1648 | 987 |

*s = Schulter

Überraschenderweise wurde gefunden, daß die erfindungsgemäßen Verbindungen der allgemeinen Formel I in, tierexperimentellen Untersuchungen in vitro und in vivo ausgeprägte antiasthmatische, antiallergische bzw. antianaphylaktische Wirkungen zeigen. Die Prüfung auf die genannten pharmakologischen Eigenschaften erfolgte z.T. nach prinzipiell aus der Literatur bekannten Meßmethoden, die in modifizierter Form zur Anwendung kamen, und zwar an der isolierten Luftröhre vom Meerschweinchen, am isolierten Lungenstreifen vom Meerschweinchen, darunter an der Arachidonsäure-induzierten Kontraktion des isolierten Lungenstreifens (vgl. Beispiel 16) und der isolierten Arteria plumonalis (vgl. Beispiel 18) sowie an der spezifisch-allergisch induzierten Bronchokonstriktion an Ovalbumin-sensibilisierten narkotisierten und künstlich beatmeten Meerschweinchen ("Meerschweinchenasthma" bzw. "Anaphylaxie des Meerschweinchens") (Vgl. Beispiel 17)/M. W. Drazen et al., J. Clin. Invest. *63*, 1 (1979); M. W. Schneider und J. M. Drazen, Amer. Rev. Resp. Dis. *121*, 835 (1980); S. S. Yen und W. Kreutner, Agents Actions *10*, 274 1980); S. S. Yen, Prostaglandins *22*, 183 (1981); Adcock, J. J.; Garland, L. G.; Brit. J. Pharmacol. *69*, 167 (1980); W. Diamantis, J. L. Melton; D. Sofia u.a., Europ. J. Pharmacol. *56* 407 (1979); P. Andersson, Brit. J. Pharmacol. *77*, 301 (1982)].

Dier erfindungsgemäßen Verbindungen der allgemeinen Formel I führen zu einer vollständigen Hemmung der Ovalbumin-induzierten Asthmareaktion bei Ovalbumin-sensibilisierten Meerschweinchen. Bemerkenswert ist, daß alle Kontrolltiere nach einmaliger Applikation von Ovalbumin i.p. (40 µg/kg Körpermasse) nach protahierend verlaufender schwerster asthmatischer Reaktion (anphylaktischer Schock?) in Apnoe starben, wogegen die mit einer der erfindungsgemäßen Substanzen (5-Methyl-2-hydroxyllaurophenon-oxim) vorbehandelten Tiere ohne jegliche bronchokonstriktorische Reaktion sogar insgesamt 3 aufeinanderfolgende Ovalbumin-Injektionen von 40 µg tolerierten. Diese Wirkung beim allergischen Asthma ist vergleichbar mit Ketotifen, einem modernen Antiasthmatikum, dessen molekularer Wirkungsmechanismus sich jedoch von dem der erfindungsgemäßen Verbindungen unterscheidet (s.u.). Die erfindungsgemäßen Verbindungen übertreffen das Ketotifen jedoch durch ein weitaus breiteres Indikationsfeld; d.h. in der Wirksamkeit auch bei nicht allergischen Formen des Asthma bronchiale. Weiterhin belegt die Hemmung der arachidonsäure-induzierten Kontraktion an der isolierten Arteria pulmonalis vom Kaninchen (Beispiel 18) die antihypertensive und spasmolytische Wirkung.

Bei dieser Methode handelt es sich um ein adequates Modell am Versuchstier für Formen des Asthma bronchiale mit Beteiligung IgE- und IgG-vermittelter allergischer Reaktionen/R. Andersson, Brit. J. Pharmacol. *77*, 301 (1982)/. Daher konnte geschlossen werden, daß die erfindungsgemäßen Verbindungen der allgemeinen Formel I auch ausgeprägte entzündungshemmende Eigenschaften aufweisen müssen. Diese Schlußfolgerung konnte an einem geeigneten tierexperimentellen Entzündungsmodell in vivo, dem carrageenin-induzierten Ödem der Rattenpfote, entsprechend der Methode nach Winter und Mitarb. [C. A. Winter, E. A. Risley and G. W. Nuss, Proc. Sco. Exp. Biol. Med. *111*, 544 (1962)] bestätigt werden (vgl. Beispiel 19).

Als molekularer Angriffspunkt für die pharmakologischen Wirkungen der erfindungsgemäßen Verbindungen wurde die Hemmung der Lipoxygenase identifiziert. Es ist bekannt, daß die durch das Enzym Lipoxygenase gebildeten Metaboliten der Arachidonsäure an der Entstehung von entzündlichen und allergischen Prozessen beteiligt sind [vgl. E. J. Goetzt, Immunology, *40*, 709 (1980); Ford-Hutchinson et al. J. Pharm. Pharmacol. *32*, 517 (1980); B. Samuelsson, Trends in Pharmacol: Sci, Mai 1980, 227; Borgeat et al., J. Med. Chem. *24*, 121 (1981)].

Die Hemmung der lipoxygenase wurde überzeugend an einer geeigneten tierischen Lipoxygenase nachgewiesen, die nach dem Verfahren von Rapoport und Mitarb. [S. M. Rapoport et al., Eur. J. Biochem. *96*, 545 (1979)] aus Kaninchenretikulozyten isoliert wurde. Die erfindungsgemäßen Verbindungen bewirkten in einer Endkonzentration von 1 mM überwiegend Hemmungen von 90% oder mehr (vgl. Beispiel 20). Die starken Hemmungen waren für einen Teil der erfindungsgemäßen Verbindungen auch bei einer Endkonzentration von 0,1 mM nachweisbar. Durch Variation der Substanzkonzentration konnten die Titrationskurven der Hemmung und daraus die Halbhemmungskonzentrationen ($I_{50}$-Werte) ermittelt werden. Sie betrug für das hoch antiasthmatisch wirksame 5-Methyl-2-hydroxy-laurophenon-oxim 7 µM. Da diese, wie auch viele der anderen erfindungsgemäßen Verbindungen aufgrund ihrer begrenzten Wasserlöslichkeit bei dieser Konzentration im in vitro-Testsystem ausfielen — sichtbar an einer deutlichen Trübung des Meßansatzes, ist zu vermuten, daß die wahren $I_{50}$-Werte weitaus geringer sind. Die Eignung der Lipoxygenase aus Kaninchenretikulozyten als Modell für den molekular-pharmakologischen Wirkort der erfindungsgemäßen Verbindungen wurde dadurch bewiesen, daß andere aus der Literatur bekannte Lipoxygenasehemmer wie z.B. 3-Amino-1-(3-trifluormethylphenyl)-pyrazolin, 5,8,11,14-Eikosatetrainsäure, 5,8,11-Eikosatriinsäure, Nordihydroguajaretsäure, Propylgallat, 4-Nitrokatechol und 3-tert.-Butyl-4-hydroxy-anisol dieses Enzym ebenfalls hoch wirksam hemmen. Die bisher bekannten Lipoxygenasehemmer erreichen jedoch nicht die antiasthmatische Wirksamkeit der erfindungsgemäßen Verbindungen. Die erfindungsgemäßen Verbindungen hemmen auch alle untersuchten aus pflanzlichen Quellen isolierten Lipoxygenasen, darunter die Sojabohnen-Lipoxygenase-1 (vgl. Beispiel 20) und die Lipoxygenase aus Erbsen. Aus diesen sowie weiteren Untersuchungen ergab sich der Schlußfolgerung, daß die erfindungsgemäßen Verbindungen universelle Hemmstoffe von Lipoxygenasen unabhängig von deren Herkunft und Positionsspezifität darstellen, woraus sich eine Reihe wertvoller pharmakologischer Eigenschaften ergibt. Von besonderem Interesse ist dabei die Beobachtung, daß das andere wichtige Enzym der Arachidonsäurekaskade, die Cyclooxygenase, die aus Samenblasen des Rindes nach dem

Verfahren von F. J. G. van der Ouderaa und M. Buytenhek, Methods in Enzymology *86*, 60 (1982), isoliert wurde, bis zu einer Endkonzentration von 1 mM nicht nennenswert gehemmt wird. In dieser Hinsicht sind die erfindungsgemäßen Verbindungen vielen bisher bekannten entzündungshemmenden Arzneimitteln überlegen, die entweder nur die Cyclooxygenase (z.B. Acetylsalicylsäure, Indometacin u.a.) oder sowohl die Lipoxygenase als auch die Cyclooxygenase (z.B. Phenidon) hemmen, woraus sich bei diesen bekannten Verbindungen unerwünschte parmakologische Nebenwirkungen ergeben (z.B. ulcerogene, gastrotoxische und proasthmatische Wirkungen der Acetylsalicylsäure).

Da für die neuen Arzneimittel die Hemmung der Lipoxygenase als molekularer Angriffspunkt identifiziert wurde, wurde auch ihr Einfluß auf die Thrombozytenaggregation untersucht. Die essentielle Rolle des Lipoxygenaseweges für die Irreversibilität der Thrombozytenaggregation ist heute bekannt [C. E. Dutilh et al. Prostaglandins and Medicine *6*, 111 (1981)]. Der Irreversibilität der Thrombozytenaggregation wird bei der Pathogenese thrombotischer Erkrankungen eine Schlüsselrolle zugeschrieben. Daher ist der Befund von außerordentlicher Bedeutung, daß die erfindungsgemäßen Verbindungen die Thrombozytenaggregation je nach Versuchsbedingungen entweder vollständig hemmen oder reversibel machen (vgl. Beispiel 21). In den Experimenten wurde die Thrombozytenaggregation entweder durch Arachidonsäure oder durch den Plättchenaktivierungsfaktor (PAF-Acether) ausgelöst.

Die genannten Experimente belegen an geeigneten biologischen Modellen die antiasthmatischen, antiallergischen, antithrombotischen und antiinflammatorischen Effekte.

Als Indikationsgebiete in der Human- und Veterinärmedizin können beispielsweise genannt werden:

1. Alle Formen des Asthma bronchiale einschließlich des infektbedingten Asthma bronchiale (intrinsic asthma), des exogen-allegischen Asthma bronchiale (extrinsic asthma) vom Typ I, II und IV nach Coombs un Gell [R. R. A. Coombs und P. G. H. Gell: The classification of allergic reactions responsible for clinical hypersensitivity and disease. In: Clinical aspects of immunology, ed. by P. G. H. Gell and R. R. A. Coombs, S. 575, Blackwell Scientific Publications, Oxford, 1968], des Analgetika-induzierten Asthma bronchiale (aspirin-induced asthma), des belastungsinduzierten Asthma bronachiale (exercise-induced asthma), des Kälteasthma, des irritativ-bedingten Asthma bronchiale und des psychogen-ausgelösten Asthma bronchiale.

2. Asthmoide Bronchitis und obstruktives Lugenemphysem sowie alle bronchokonstriktorischen Zustände, die als Begleitsymptom anderer Erkrankungen oder Nebenwirkung medizinischer Maßnahmen, z.B. Narkosekomplikationen oder bronchopastische Reaktionen nach Applikation beta-adrenerger Blockersubstanzen, auftreten.

3. Allergische Erkrankungen im weiteren Sinne, insbesondere:
— atopische Dermatitis
— allergische Rhinitis (saisonale Rhinitis, pereniale Rhinitis aber auch vasomotorische Rhinitis)
— Urticaria
— Angiooedem
— Kontaktdermatitis (Kontaktekzem)
— allergische Erkrankungen des Gastrointestinaltraktes
— allergische Conjunctivitis.

4. Alle Formen der Thrombose, sowohl zur Behandlung bestehender Thrombose (Thrombophlebitis) als auch zur Thromboseprophylaxe bei
— chronisch-ischämischer Herzkrankheit
— Nachbehandlung bei Myokardinfarkt
— chronisch rezidivierende Thrombose
— chronische Thrombophlebitis

5. Der Einsatz als nichtsteroidale Antiphlogistika, wobei die Verbindungen der Formel I vor allem bei solchen entzündlichen Prozessen indiziert sind, bei denen die herkömmlichen Antiphlogistika (z.B. Acetylsalicylsäure, Salicylat u.a.), die einen Angriffspunkt außerhalb der Lipoxygenase aufweisen, ungenügende therapeutische Effekte zeigen, insbesondere bei purulenten Entzündungen und bei rheumatischen und arthritischen Erkrankungen.

6. Alle Formen des arteriellen Hochdrucks, insbesondere auch des pulmonalen Hochdrucks (im Lungenkreislauf).

7. Spastische Zustände der glatten Muskulatur verschiedener Genese, insbesondere in verschiedenen Abschnitten des Verdauungs- und Urogenitaltraktes sowie der Blutgefäßmuskulatur.

Aufgrund anderer aus der Literatur bekannten pharmakologischen Wirkungen von Lipoxygenasehemmern lassen sich für die erfindungsgemäßen Verbindungen der Formel I auch antiatherosklerotische, gastroprotektive sowie antimetatastatische Wirkungen ableiten.

Die Verbindungen der allgemeinen Formel I eignen sich als Wirkstoffe für oral, perlingual, rektal, parenteral, intravenös oder perkutan sowie als Aerosol anwendbare Arzneimittel zur Behandlung von verschiedenen Formen des Asthma bronchiale sowie von Thrombose, rheumatischen, arthritischen und anderen entzündlichen Krankheiten.

Aufgrund ihrer besonders günstigen pharmakologischen Eigenschaften sind zu nennen:
5-Methyl-2-hydroxy-laurophenon-oxim
4-Methyl-2-hydroxy-caprophenon-oxim
5-Methyl-2-hydroxy-caprophenon-oxim

4-Decyloxy-2-hydroxy-acetophenon-oxim
4-Decyloxy-2-hydroxy-propiophenon-oxim
4-Benzyloxy-2-hydroxy-acetophenon-oxim
4-Decyloxy-2-hydroxy-caprophenon-oxim
4-Hexyloxy-2-hydroxy-acetophenon-oxim
4-Pentoxy-2-hydroxy-acetophenon-oxim
4-Pentoxy-2-hydroxy-caprophenon-oxim
4-Butoxy-5-n-hexyl-2-hydroxy-acetophenon-oxim
5-Methyl-2-hydroxy-caprophenon-(N-phenyl-carbamoyl)-oxim

Zur vorliegenden Erfindung gehören pharmazeutische Zubereitungen, die neben nicht toxischen, inerten pharmazeutisch geeigneten Trägerstoffen einen oder mehrere erfindungsgemäße Wirkstoffe enthalten oder die aus einem oder mehreren erfindungsgemäßen Wirkstoffen bestehen.

Unter nicht toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Sirupe, Suppositorien, Lösungen, Suspensionen und Emulsionen, Pasten, Salben, Gele, Cremes, Lotions, Puder, Sprays und Aerosole genannt.

Tabletten, Dragees, Kapseln, Pillen und Granulate können den oder die Wirkstoffe neben den üblichen Trägerstoffen enthalten; wie a) Füll- und Streckmittel, z.B. Stärken, Milchzucker, Rohrzucker, Glucose, Mannit und Kieselsäure, b) Bindemittel, z.B. Carboxymethylcellulose, Alginate, Gelatine, Polyvinylpyrrolidon, c) Feuchthaltemittel, z.B. Glycerin, d) Sprengmittel, z.B. Agar-Agar, Calciumcarbonat und Natriumbicarbonat, e) Lösungsverzögerer, z.B. Paraffin und f) Resorptionsbeschleuniger, z.B. quartäre Ammoniumverbindungen, g) Netzmittel, z.B. Cetylalkohol, Glycerinmonostearat, h) Adsorptionsmittel, z.B. Kaolin und Bentonit und i) Gleitmittel, z.B. Talkum, Calcium- und Magnesiumstearat, Natriumlaurylsulfat und feste Polyethylenglycole oder Gemische der unter a) bis i) aufgeführten Stoffe.

Die Tabletten, Dragees, Kapseln, Pillen und Granulate können mit den üblichen gegebenenfalls Opalisierungsmittel enthaltenden Überzügen versehen sein und auch so zusammengesetzt sein, daß sie den oder die Wirkstoffe nur oder bevorzugt in einem bestimmten Teil des Intestinaltraktes, gegebenenfalls verzögert angeben, wobei als Einbettungsmassen z.B. Polymersubstanzen und Wachse verwendet werden können.

Der oder die Wirkstoffe können gegebenenfalls mit einem oder mehreren der eben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, z.B. Polyethylenglycole, Fette, z.B. Kakaofett und höhere Ester (z.B. $C_{14}$-Alkohol mit $C_{16}$-Fettsäure/oder Gemische dieser Stoffe).

Salben, Pasten, Cremes und Gele können neben dem oden den Wirkstoffen die üblichen Trägerstoffe enthalten, z.B. tierische und pflanzliche Fette, Wachse, Paraffine (z.B. Erdölfraktionen), Stärke, Tragant, Cellulosederivate, Polyethylenglycole, Silicone, Bentonite, Talkum, Kieselsäure und Zinkoxid oder Gemische dieser Stoffe.

Sprays und Puder können neben dem oder den Wirkstoffen die üblichen Trägerstoffe enthalten, z.B. Milschzucker, Talkum, Kieselsäure, Aluminiumhydroxid, Calciumsilikat und Polyamidpulver oder Gemische dieser Stoffe. Sprays können zusätzlich die üblichen Treibmittel, z.B. Chlorfluorkohlenwasserstoffe, enthalten.

Lösungen und Emulsionen können neben dem oder den Wirkstoffen die üblichen Trägerstoffe wie nicht toxische organische Lösungsmittel, Lösungsvermittler und Emulgatoren, z.B. Wasser, Dimethylsulfoxid, Ethylalkohol, Isopropylalkohol, Methylglycol, Ethylcarbonat, Ethylacetat, Benzylalkohol, Benzylbenzoat, Propylenglycol, 1,3-Butylenglycol, Dimethylformamid, Öle, insbesondere Baumwollsaatöl, Erdnußöl, Cashewnußöl, Maiskeimöl, Olivenöl, Ricinusöl und Sesamöl, Glycerin, Glycerinformal, Tetrahydrofurfurylalkohol, Polyethylenglycole und Fettsäureester des Sorbitans oder Gemische dieser Stoffe enthalten.

Zur parentalen Applikation können die Lösungen und Emulsionen auch in steriler und blutisotonischer Form vorliegen. Suspensionen können neben dem oder den Wirkstoffen die üblichen Trägerstoffe wie flüssige Verdünnungsmittel, z.B. Wasser, Dimethylsulfoxid, Ethylalkohol, Propylenglycol, Suspendiermittel, z.B. ethoxylierte Isostearylalkohole, Polyoxyethylensorbit- und Sorbitanester, mikrokristalline Cellulose, Aluminiummetahydroxid, Bentonit, Agar-Agar oder Tragant oder Gemische dieser Stoffe enthalten.

Für die Formulierung können auch Dispergiermittel (z.B. Lignin, Sulfitablaugen, Methylcellulose, Stärke un Polyvinylpyrrolidon) verwendet werden.

Die genannten Formulierungsformen können auch Färbemittel, Konservierungsstoffe sowie geruchs- und geschmacksverbessernde Zusätze, z.B. Pfefferminzöl und Eucalyptusöl und Süßmittel, z.B. Saccharin, enthalten.

Die therapeutisch wirksamen Verbindungen sollen in den oben aufgeführten pharmazeutischen Zubereitungen vorzugsweise in einer Konzentration von etwa 0,1 bis 99,5 vorzugsweise von etwa 0,5 bis 90 Masse-% der Gesamtmischung vorhanden sein, d.h. in Mengen, die ausreichend sind, um den angegebenen Dosierungsspeilraum zu errichen.

Die oben ausgeführten pharmazeutischen Zubereitungen können außer den erfindungsgemäßen Wirkstoffen auch weitere pharmazeutische Wirkstoffe enthalten, z.B. Antihistaminika und Cyclooxygenasehemmer.

Die Herstellung der oben aufgeführten pharmazeutischen Zubereitungen erfolgt in üblicher Weise nach bekannten Methoden, z.B. durch Mischen der Wirkstoffe mit den Trägerstoffen.

Zur vorliegenden Erfindung gehöhrt auch die Verwendung der erfindungsgemäßen Wirkstoffe sowie der pharmazeutischen Zubereitungen, die einen oder mehrere Wirkstoffe enthalten, in der Human- und Veterinärmedizin zur Verhütung, Besserung und/oder Heilung der oben angeführten Krankheiten.

Die Wirkstoffe oder die pharmazeutischen Zubereitungen können lokal, oral, parenteral, intraperitoneal und/oder rektal, vorzugsweise oral, insbesondere als Aerosol appliziert werden.

Im allgemeinen hat es sich als vorteilhaft erweisen, den oder die erfindungsgemäßen Wirkstoffe in Gesamtmengen von etwa 0,05 bis etwa 100, vorzugsweise 0,1 bis 50 mg/kg Körpermasse je 24 Stunden, gegebenenfalls in Form mehrerer Einzelgaben zur Erzielung der gewünschten Ergebnisse zu verabreichen.

Es kann jedoch erforderlich sein, von den genannten Dosierungen abzuweichen, und zwar in Abhängigkeit von der Art und dem Körpergewicht des zu behandelnden Objektes, der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der oben genannten Wirkstoffmenge auszukommen, während in anderen Fällen die oben angeführte Menge Wirkstoff überschritten werden muß.

Die nachfolgenden Beispiele erläutern die Erfindung näher, sollen jedoch ihren Umfang in keiner Weise beschränken.

Ausführungsbeispiele

## Beispiel 1

4-Methyl-2-hydroxy-caprophenon-oxim

m-Kresol und Caproylchlorid werden in bekannter Weise zum Ester umgesetzt, der nach Fries zum 4-Methyl-2-hydroxy-caprophenon umgelagert wird (P. P. T. Sah und H. H. Anderson, J. Am. Chem. Soc. *63*, 3164 (1941). 20 g (0.1, Mol) des Ketons, 10,6 g (0,15 Mol) Hydroxylaminhydrochlorid, 35 ml Ethanol und 7 ml Wasser werden unter Schütteln mit 19,5 g (0,5 Mol) Natriumhydroxid versetzt. Das Reaktionsgemisch erhitzt man 5 min unter Rückflußkühlung, läßt erkalten und gibt das Gemisch in eine wässrige HCl-Lösung (55 ml konz. HCl und 350 ml Wasser). Das anfallende Oxim wird in Ether aufgenommen und mit wasserfreiem Natriumsulfat getrocknet. Nach Abdestillieren des Ethers erhält man 21 g Rohprodukt. Durch Umkristallisation aus Petrolether wird as Oxim mit einem Fp. von 45°C erhalten.

## Beispiel 2

5-Methyl-2-hydroxy-caprophenon-oxim

p-Kresol und Capropylchlorid werden in bekannter Weise zum Ester umgesetzt, der nach Fries zum 5-Methyl-2-hydroxycaprophenon vom $Kp_{65}$155° umgelagert wird (vgl. P. P. T. Sah u. H. H. Anderson, J. Am. Chem. Soc., *63*, 3164 (1941). 20 g (0,1 Mol) des Ketons wurden in 45 ml Ethanol gelöst und mit einer konzentrierten wässrigen Lösung von 7,5 g Hydroxylaminhydrochlorid und 15 g kristallinem Natriumacetat 30 min unter Rückflußkühlung zum Sieden erhitzt. Danach wird das Gemisch mit Wasser versetzt und das abgeschiedene Oxim aus Ethanol/Wasser umkristallisiert, Fp. 83—85°C.

## Beispiel 3

5-Methyl-2-hydroxy-caprophenon-(N-phenyl-carbamoyl)-oxim

2,2 g (0,02 Mol) 5-Methyl-2-hydroxy-caprophenon-oxim werden in 25 ml Toluen, gegebenenfalls unter leichtem Erwärmen, gelöst und unter Umschwenken langsam 1,2 g (0,01 Mol) Phenylisocyanat zugesetzt. Nach 1 stündigem Stehen wird das Lösungsmittel im Vakuum verdampft, wobei der Rückstand kristallisiert. Durch Umkristallisation aus Ethanol oder Ethanol/Wasser werden farblose Kristalle vom Fp. 117—120°C erhalten.

## Beispiel 4

5-Methyl-2-hydroxy-laurophenon-oxim

Der aus p-Kresol und Lauroylchlorid gebildete Ester vom Fp 28°C wird in bekannter Weise durch Fries-Umlagerung in das Keton vom Fp 42—43°C überführt. 29 g (0,1 Mol) des Ketons werden in soviel Ethanol gelöst, daß bei Zugabe einer wässrigen Lösung von 7 g Hydroxylamin-hydrochlorid und 15 g kristallinem Natriumacetat eine klare Lösung entsteht, die unter Rückflußkühlung 20 min erhitzt wird. Auf der Reaktionslösung scheidet sich das Oxim durch Kuhlen oder Versetzen mit Wasser ab. Aus Ethanol farblose Kristalle vom Fp 93°C.

## Beispiel 5

3-Chlor-2-hydroxy-caprophenon-oxim

o-Chlorphenol und Caproylchlorid werden in bekannter Weise zum Ester umgesetzt, der nach Fries mit $AlCl_3$ durch 1 stündiges Erhitzen auf 120°C zum 3-Chlor-2-hydroxy-caprophenon vom Fp. 78—80°C (Ethanol/Wasser, n-Heptan) umgelagert wird. 22,6 g (0,1 Mol) des Ketons werden mit 15,2 (0,22 Mol) Hydroxylaminhydrochlorid in 150 ml absol. Ethanol und 25 ml Pyridin unter Rückflußkühlung 3 Stunden gekocht. Nach Verdampfen der Lösungsmittel im Vakuum verbleibt ein öliger Rückstand. Durch Behandeln

mit Ethanol/Wasser und Umkristallisieren aus n-Heptan erhält man das Oxim vom Fp. 102—103°C.

Analog wurden die folgenden Beispiele erhalten:
5-Chlor-2-hydroxy-caprophenon-oxim, Fp. 100°C (n-Heptan)
3-Chlor-2-hydroxy-laurophenon-oxim, Fp. 96°C (Ethanol)
5-Chlor-2-hydroxy-laurophenon-oxim, Fp. 97°C (Ethanol).

Beispiel 6

4-Pentoxy-2-hydroxy-acetophenon-oxim

30,4 g (0,2 Mol) 2,4-Dihydroxyacetophenon wurden mit 45 g (0,3 Mol) n-Pentylbromid und 28 g wasserfreiem Kaliumcarbonat in 300 ml getrocknetem Aceton 24 Stunden unter Rückflußkühlung gekocht, das Aceton im Vakuum verdampft, der Rückstand mit wenig Wasser versetzt und dann ausgeethert. Aus der mit wasserfreiem $Na_2SO_4$ getrockneten etherischen Phase wurde nach Entfernen des Ethers das Keton vom Fp. 33°C erhalten. Das Reaktionsgemisch aus 11,1 g (0,05 Mol) Keton, 7,5 g Kaliumacetat und 4,5 g Hydroxylaminhydrochlorid wurde dann 3 Stunden unter Rückflußkühlung erhitzt, heiß filtriert und das Filtrat zur Abscheidung des Oxims mit Wasser versetzt. Aus n-Heptan wurden 9 g Oxim vom Fp. 66—67°C erhalten.

Analog wurden die folgenden Beispiele erhalten:
4-Butoxy-2-hydroxy-acetophenon-oxim, Fp. 87°C (Ethanol/Wasser und n-Heptan)
4-Dodecyloxy-2-hydroxy-propiophenon-oxim, Fp. 63°C (n-Heptan)
4-Hexadecyloxy-2-hydroxy-acetophenon-oxim, Fp. 86—87°C (n-Heptan)
4-Octadecyloxy-2-hydroxy-acetophenon-oxim, Fp. 90°C (Ethanol).

Beispiel 7

4-Decyloxy-2-hydroxy-acetophenon-oxim

30,4 g (0,2 Mol) 2,4-Dihydroxyaceophenon wurden mit 66,3 g (0,3 Mol) n-Decylbromid und 28 g wasserfreiem Kaliumcarbonat in 300 ml getrocknetem Aceton 22 Stunden auf dem Wasserbad unter Rükflußkühlung gekocht und das Aceton im Vakuum verdampft. Der Rückstand wird in wenig Wasser aufgenommen, mit Ether extrahiert und die abgetrennte organische Phase mit wasserfreiem $Na_2SO_4$ getrocknet. Nach Abdestillieren des Ethers wird aus dem Rückstand nach Umkristallisieren aus Ethanol das Keton vom Fp. 35°C erhalten. Das Reaktionsgemisch aus 14,6 g (0,05 Mol) Keton und 7,6 g (0,11 Mol) Hydroxylaminhydrochlorid in 90 ml absol. Ethanol und 30 ml Pyridin wurde 3 Stunden unter Rückflußkühlung gekocht. Nach Abdampfen der Lösungsmittel im Vakuum wurde der Rückstand zuerst aus Ethanol/Wasser, dann aus n-Heptan zu farblosen Kristallen vom Fp. 69—70°C umkristallisiert.

Beispiel 8

4-Decyloxy-2-hydroxy-propiophenon-oxim

16,6 g (0,1 Mol) 2,4-Dihydroxypropiophenon wurden mit 33,1 g (0,15 Mol) Decylbromid und 14 g wasserfreiem Kaliumcarbonat in 150 ml getrocknetem Aceton 20 Stunden unter Rückflußkühlung erhitzt und das Aceton im Vakuum verdampft. Der Rückstand wird in Wasser aufgenommen, mit Ether ausgeschüttelt und die organische Phase mit wasserfreiem $Na_2SO_4$ getrocknet. Nach Abdestillieren des Ethers wurde das Keton vom Fp. 30°C erhalten. Das Reaktionsgemisch aus 9,2 g (0,03 Mol) des Ketons in 15 ml Pyridin und 25 ml absol. Ethanol mit 4,5 g (0,066 Mol) Hydroxylaminhydrochlorid wurde 3 Stunden unter Rückflußkühlung gekocht. Nach Abdampfen der Lösungsmittel im Vakuum wird der Rückstand mit Wasser gewaschen und aus Ethanol umkristallisiert. Farblose Kristalle vom Fp. 58—59°C.

Beispiel 9

4-Benzyloxy-2-hydroxy-acetophenon-oxim

30 g (0,2 Mol) 2,4-Dihydroxyacetophenon wurden in 300 ml getrocknetem Aceton gelöst, 28 g wasserfreies Kaliumcarbonat und danach 38 g (0,3 Mol) Benzylchlorid zugegeben und die Mischung ca. 20 Stunden unter Rückflußkühlung erhitzt. Nach Abdestillieren des Acetons versetzt man den Rückstand mit $2nH_2SO_4$, saugt das feste Keton ab, behandelt es mit 2 n KOH und wäscht dann mit Wasser neutral. Aus Ethanol erhält man 22 g farblose Kristalle vom Fp. 103—105°C, die mit 12,8 g Hydroxylaminhydrochlorid und 18,8 g Kaliumacetat in 110 ml Ethanol 3 Stunden unter Rückflußkühlung gekocht wurden. Nach Filtrieren des Reaktionsgemisches erhält man durch Zugabe von Wasser die Titelverbindung, die aus Ethanol/Wasser einen Fp. von 143°C zeigt.

Beispiel 10

4-Butoxy-5-n-hexyl-2-hydroxy-acetophenon-oxim

Ein Gemisch aus 7,1 g (0,03 Mol) 2,4-Dihydroxy-5-n-hexylacetophenon, 6,1 g (0,045 Mol) n-Butylbromid und 4,2 g wasserfreiem Kaliumcarbonat wurde in 50 ml getrocknetem Aceton auf dem Wasserbad unter Rückflußkühlung 20 Stunden gekocht, das Lösungsmittel abdestilliert, der Rückstand in Wasser aufgenommen, mit Ether ausgeschüttelt und die organische Phase mit wasserfreiem $Na_2SO_4$ getrocknet. Nach Abtrennen des Ethers wurde das Keton vom Fp. 37°C (n-Heptan) erhalten. 2,8 g (0,01 Mol) des Ketons wurden in 30 ml absol. Ethanol und 6 ml Pyridin mit 1,52 g (0,022 Mol) Hydroxylamin-

hydrochlorid 3 Stunden unter Rückflußkühlung gekocht. Nach Abdestillieren der Lösungsmittel und Waschen mit Wasser wurde der Rückstand aus n-Heptan umkristallisiert, Fp. 62°C.

Beispiel 11

4-Pentoxy-2-hydroxy-caprophenon-oxim

20,8 g (0,1 Mol) 2,4-Dihydroxycaprophenon werden mit 22 g (0,145 Mol) n-Pentylbromid und 14 g wasserfreiem $K_2CO_3$ in 150 ml getrocknetem Aceton unter Rückfluß auf dem Wasserbad 30 Stunden bei 60—70°C gehalten. Das Aceton wird dann im Vakuum verdampft und der Rückstand mit Wasser versetzt. Nach dem Ausethern, Trocknen der organischen Phase mit $Na_2SO_4$ und Entfernen des Ethers verbleibt ein Rückstand, der beim Kühlen erstarrt. Durch Umkristallisieren aus Ethanol erhält man das Keton mit einem Fp. von 41—42°C. 14 g (0,05 Mol) des Ketons wurden mit 7,6 g (0,11 Mol) Hydroxylaminhydrochlorid und 15 g Kaliumacetat in 200 ml Ethanol 3 Stunden unter Rückflußkühlung gekocht. Nach dem Abdestillieren des Ethanols wurde mit ca. 100 ml warmem Wasser versetzt und das als unlöslicher Rückstand verbliebene Oxim aus Ethanol/Wasser zu farblosen Kristallen vom Fp. 80°C umkristallisiert.

Beispiel 12

4-Decyloxy-2-hydroxy-caprophenon-oxim

20,8 g (0,1 Mol) 2,4-Dihydroxycaprophenon werden mit 32 g (0,145 Mol) n-Decylbromid und 14 g wasserfreiem $K_2CO_3$ in 150 ml getrocknetem Aceton wie im Beispiel 11 zum Keton vom Fp. 45—47°C umgesetzt, das mit Hydroxylaminhydrochlorid in Abwesenheit von Kaliumacetat in ethanolischer Lösung zum Oxim vom Fp. 57°C (Ethanol/Wasser) reagiert.

Beispiel 13

4-Octyloxy-2-hydroxy-laurophenon-oxim

29,2 g (0,1 Mol) 2,4-Dihydroxylaurophenon werden mit 28 g (0,145 Mol) n-Octylbromid und 14 g wasserfreiem $K_2CO_3$ in 150 ml getrocknetem Aceton wie im Beispiel 11 zum Keton vom Fp. 56—57°C (Ethanol) und dann mit Hydroxylaminhydrochlorid in Anwesenheit von Kaliumacetat in ethanolischer Lösung zum Oxim vom Fp. 60—63°C (Ethanol, n-Heptan) umgesetzt.

Analog wurde das folgende Beispiel erhalten: 4-Decyloxy-2-hydroxy-laurophenon-oxim, Fp. 64—69°C (n-Heptan; Ethanol).

Beispiel 14

4-Cyclohexyloxy-2-hydroxy-propiophenon-oxim

8,3 g (0,05 Mol) 2,4-Dihydroxypropiophenon werden mit 12 g (0,07 Mol) Cyclohexylbromid und 7 g wasserfreiem $K_2CO_3$ in 50 ml Ethylglykol unter Rückflußkühlung 8 Stunden bei Siedetemperatur gehalten. Danach wird das Reaktionsgemisch filtreirt und nach dem Abkühlen mit 250 ml Wasser versetzt. Das erhaltene Keton zeigt nach dem Umkristallisieren aus Ethanol einen Fp. von 80—81°C. Anschließend wurde das Keton mit Hydroxylaminhydrochlorid in Anwesenheit von Kaliumacetat in ethanolischer Lösung zum Oxim vom Fp. 65—69°C (Ethanol, n-Heptan) umgesetzt.

Beispiel 15

Wirkung von 5-Methyl-2-hydroxy-laurophenon-oxim auf die Carbachol-induzierte Kontraktion der isolierten Meerschweinchentrachea

Die Prüfung der Verbindung auf antiasthmatische Aktivität erfolgte an der isolierten Trachealkette von Meerschweinchen nach aus der Literatur (siehe S. 6) bekannten Meßmethoden in modifizierter Form. Die Messungen erfolgten im thermostatierten Organbad isoton unter Verwendung einer Kontraktionsmeßvorrichtung mit Hebelaufnehmer, Meßspule, Meßverstärker (induktive Messung mit Hilfe eines Hochfrequenzschwingkreises). Die Begasung erfolgte mit Luft. Die Suspensionslösung hatte folgende Zusammensetzung: 39,46 g NaCl, 2,2 g KCl, 6,07 g Tris, 1,0 g $CaCl_2$, 9,9 g Glukose, 1,0 ml gesättigte $MgCl_2$-Lösung, 43 ml 1N HCl pro 5 l, pH 7,4.

Der Spasmus wurde durch 3,9 μM Carbachol ausgelöst. Nachträglicher Zusatz des untersuchten Wirkstoffes verursachte eine starke Dilatation, die bereits bei einer Wirkstoffkonzentration von 50 μM deutlich nachweisbar war.

Beispiel 16

Wirkung von 5-Methyl-2-hydroxy-laurophenon-oxim auf die durch exogene Arachidonsäure ausgelöste Kontraktion des isolierten Meerschweinchenlungenstreifens

Die experimentelle Anordnung dieses Testsystems war analog zu Beispiel 15. Als Testobjekt dienten jedoch isolierte Lungenstreifen des Meerschweinchens. Die Kontraktion wurde hierbei durch ansteigende Konzentrationen von Arachidonsäure (konzentrierte Lösung in Ethanol, in $N_2$-Atmosphäre gelagert) ausgelöst und kumulativ gemessen.

10 μM der o.g. erfindungsgemäßen Verbindung führten zu einer Reduktion der Kontaktionsantwort auf Arachidonsäure im Bereich von 0,1—100 μM um 70% ("Metactoide Hemmung" nach "General Theory of Drug-Receptor-Interactions" von F. G. van den Brink in Kinetics of Drug Action ed. by J. M. van Ronum, Springer Berlin, Heidelberg, New York, 1977, Kap. 4, S. 169—254).

Beispiel 17

Wirkung auf die Allergen-induzierte Bronchokonstriktion am sensibilisierten Meerschweinchen in vivo ("allergisches Meerschweinchenasthma")

Die Untersuchung erfolgte an männl. Meerschweinchen, 30—35 Tage nach Sensibilisierung mit 330 mg $Al(OH)_3$ und 33 µg Ovalbumin pro kg Körpermasse (frisch zubereitet in physiol. NaCl-Lösung).

[Methode modifiziert nach P. Andersson, Brit. J. Pharmacol. *77*, 301 (1982)]. Bei den Tieren wurde die Narkose mit Urethan (1,3 g/kg Körpermasse i.p.), die Muskelrelaxation mit Pavulon® (2 mg/kg Körpermasse i.v.) vorgenommen. Die allergen-induzierte Bronchokonstriktion, die sich bei allen Kontrolltieren innerhalb weniger Sekunden zum vollbild des anaphylaktischen Schocks entwickelte, wurde mit 40 µg Ovalbumin i.v. ausgelöst. Die tracheostomierten und venenkatheterisierten Tiere wurden in einem Tankrespirator mit einem rhythmischen Unterdruck beatmet (f = 16 $min^{-1}$; Inspiration: Exspiration = 1:1). Pneumotachograpisch wurden Gasstrom ($\dot{V}$) und Atemzugvolumen ($V_T$) sowie das EKG kontrolliert. Die Vorbehandlung der Tiere erfolgte mit 2 Injektionen i.p. von fein gemörsertem 5-Methyl-2-hydroxyl-laurophenon-oxim, aufgeschlämmt in Agar-Agar, in Dosen von je 10 mg/kg Körpermasse 90 und 60 Minuten vor Versuchsbeginn.

Die Substanz führte bei allen untersuchten Tieren ausnahmslos zu einer kompletten Hemmung der Ovalbumin-induzierten Asthmareaktion. Alle Kontrolltiere hingegen starben nach einmaliger Applikation von 40 µg Ovalbumin i.v. nach protrahierend verlaufener schwerster asthmatischer Reaktion in Apnoe ("silence chest syndrome").

Die vorbehandelten Tiere tolerierten reaktionslos sogar 2 weitere aufeinanderfolgende Ovalbuminjektionen von je 40 µg/kg Körpermasse. Vergleichbare antiasthmatische, antiallergische bzw. antianaphylaktische Wirkungen am Ganztiermodell sind auch von modernen Antiasthmatika wie Ketotifen und Dinatriumcromoglykat nicht bekannt (C. Armour u. D. M. Temple, Agents Actions *12*, 285 [1982]).

Beispiel 18

Wirkung von 5-Methyl-2-hydroxy-laurophenon-oxim auf die durch exogene Arachidonsäure ausgelöste Kontraktion der isolierten Pulmonalarterie vom Kaninchen

Die experimentelle Anordnung dieses Testsystems entspricht Beispiel 15.

Als Testobjekt dienten isolierte Streifen der Lungenarterie von Kaninchen.

Die Kontraktion wurde in Anwesenheit von 10 µM Indometazin (Blockierung des Cyclooxygenasewegs der Arachidonsäureverwertung) durch ansteigende Konzentrationen von Arachidonsäure ausgelöst und kumulativ gemessen.

10 µM 5-Methyl-2-hydroxy-laurophenon-oxim führten zu einer Reduktion der Kontraktionsantwort auf Arachidonsäure im Bereich von 0,1—100 µM um 60 bis 80% ("Metactoide Hemmung" nach "General Theory of Drug-Receptor-Interactions" von F. G. van den Brink in Kinetics of Drug Action ed. by I. M. van Rossum, Springer Berlin, Heidelberg, New York, 1977, Kap. 4, S. 169—254).

Beispiel 19

Hemmung des Carrageeninödems der Rattenpfote

Das Carrageeninödem wird in der internationalen Literatur als Modellsystem für entzündungsauslösende (prophlogistische) Prozesse benutzt und bietet die Möglichkeit der in vivo-Testung von Substanzen auf entzündungshemmende (antiphlogistische) Aktivität. Die Testung erfolgte nach der international üblichen Methodik [C. A. Winter, E. A. Risley and G. W. Nuss, Proc. Soc. Exp. Biol. Med. *111*, 544 (1962)]. 5-Methyl-2-hydroxy-laurophenon-oxim wurde 10 Ratten i.p. in einer Dosis von 50 mg/kg Körpermasse bei gleichzeitiger Gabe von 0,1 ml 0,1 %iger Carrageeninlösung pro Tier appliziert. Das Ausmaß des Pfotenödems wurde nach der Applikation stündlich gemessen und mit der Kontrollgruppe verglichen. Dabei ergaben sich folgende Ergebnisse:

TABELLE 2

Hemmung des Carrageeninödems durch 5-Methyl-2-hydroxy-laurophenon-oxim

| Zeit (h) | Hemmung (%) |
|---|---|
| 0,5 | 43* |
| 1 | 47* |
| 2 | 34* |
| 3 | 34* |
| 4 | 31 |
| 5 | 29 |

*signifikant mit $P < 0,05$

Beispiel 20

Hemmung der Aktivität der Lipoxygenase aus Kaninchenretikulozyten

Die Lipoxygenase aus Kaninchenretikulozyten wurde nach dem in der Literatur beschriebenen Verfahren in elektrophoretisch und immunologisch reiner Form erhalten [S. M. Rapoport et al., Eur. J. Biochem. *96*, 545 (1979)]. Die Bestimmung der Lipoxygenaseaktivität erfolgte bei 25°C über die amperometrische messung des $O_2$-Verbrauchs mittels einer Clarck-Elektrode in folgendem System: 0,1 M Kaliumphosphat pH 7.4 mit 0,2% Natriumcholat und 0,53 mM Linolsäure. Die Enzymkonzentration betrug im Meßansatz 25 nM. Die zu prüfenden Substanzen wurden in Methylglykol (frisch destilliert im Vakuum) gelöst und 10 min bei der Meßtemperatur mit dem Enzym in Abwesenheit von Natriumcholat und Linolsäure vorinkubiert. Die Verdünnungen der Verbindungen wurden so gewählt, daß die Endkonzentrationen an Methylglykol im Vorinkubationsansatz 2% nicht überstieg; unter diesen Bedingungen traten keine nennenswerten Hemmungen in den Kontrollansätzen auf. Die Enzymreaktion wurde durch Zusatz von Natriumcholat und Linolsäure gestartet. Durch Variation der Wirkstoffkonzentration wurden die Titrationskurve der Hemmung und daraus die erforderlichen Konzentrationen für eine 50 %ige Hemmung ermittelt. Zum Vergleich wurden die Verbindungen auch an handelsüblicher Lipoxygenase-1 aus Sojabohnen getestet.

Im Gegensatz zu den erfindungsgemäßen Verbindungen erwiesen sich die modernen Antiasthmatika Ketotifen und Dinatriumcromoglykat auf die Retikulozytenlipoxygenase selbst in einer Endkonzentration von 1 mM als unwirksam.

TABELLE 3

Hemmung der Lipoxygenasen aus Kaninchenretikulozyten und Sojabohnen

| Verbindung | Endkonzentration (mM) | Hemmung (%) der Lipoxygenase aus: Retikulozyten (in Klammern $I_{50}$-Wert in mM) | Hemmung (%) der Lipoxygenase aus: Sojabohnen |
|---|---|---|---|
| 5-Methyl-2-hydroxy-laurophenon-oxim | 1,0 | 100(0,007) | 67 |
| 5-Chlor-2-hydroxy-caprophenon-oxim | 1,0 | 90(0,3) | 24 |
| 4-Hexyloxy-2-hydroxy-acetophenon-oxim | 1,0 | 100(0,26) | 68 |
| 4-Hexadecyloxy-2-hydroxy-acetophenon-oxim | 0,5 | 75(0,5) | 38 |

Beispiel 21

Hemmung der Arachidonsäure- oder PAF-induzierten Thrombozytenaggregation

Die Prüfung der Verbindungen auf antithrombotische und thrombolytische Aktivität erfolgte am authentischen Zellsystem des Menschen in vitro. Thrombozytenreiches Plasma aus dem Blut gesunder Spender wurde durch Zentrifugation bei 1000 × g erhalten. Die Messung der Thrombozytenaggregation erfolgte mittels eines Aggregometers aufgrund der diffusen Lichtstreuung bzw. der Lichtabsorption der entstehenden Zellaggregate. Das thrombozytenreiche Plasma wurde bei 37°C 3 min mit den Wirkstoffen vorinkubiert; danach wurde die Thrombozytenaggregation durch Zusatz von entweder 0,8 mM Arachidonsäure oder 1 µM Plättchenaktivierungsfaktor (PAF-Acether) ausgelöst. Die Ansätze wurden dabei mit einer Geschwindigkeit von 800 Umdrehungen/min gerührt.

Je nach der verwendeten Wirkstoffkonzentration trat entweder eine starke Verzögerung oder eine vollständige Hemmung der Thrombozytenaggregation ein.

Bei der durch PAF-Acether ausgelösten Aggregation bewirkten alle untersuchten Verbindungen in einer Konzentration von 40 µM eine Auflösung der zunächst gebildeten Zellaggregate. Identische Effekte wurden beobachtet, wenn in gewaschenen Thrombozytensuspensionen die Aggregation mit 16 µM Arachidonsäure ausgelöst wurde. Aus diesem Verhalten geht hervor, daß die untersuchten Lipoxygenasehemmer die Thrombozytenaggregation in ihrer irreversiblen Phase blockieren und dadurch thrombolytisch aktiv sind.

So bewirkte z.B. 4-Hexyloxy-2-hydroxy-acetophenon-oxim bei einer Konzentration von 33 µM eine Verzögerung der Aggregation um ca. 2 min, während 60 µM eine vollständige Hemmung verursachten. Ähnliche Ergebnisse wurde auch mit anderen erfindungsgemäßen Verbindungen sowie mit bekannten Lipoxygenasehemmern, wie z.B. 4-Nitrokatechol, erzielt.

**Patentansprüche für die Vertragsstaaten: BE CH DE FR GB IT LI NL SE**

1. Pharmazeutische Zusammensetzungen, gekennzeichnet durch einen Gehalt an 1-(2-Hydroxyaryl)-alkan-1-on-oximen der allgemeinen Formel (I)

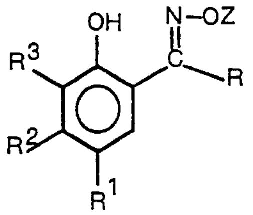

I

als Wirkstoff, worin bedeuten:

a) R $C_{11-18}$-Alkyl für $R^2$, $R^3$ und Z gleich Wasserstoff $R^1$ Wasserstoff, $C_{1-12}$-Alkyl oder Chlor oder

b) R $C_{1-18}$-Alkyl oder Aralkyl für $R^2$ $C_{1-12}$-Alkyl oder $OR^4$, wo $R^4$ für $C_{1-18}$-Alkyl, $C_{3-8}$-Cycloalkyl oder Aralkyl steht

$R^3$ $C_{1-12}$-Alkyl oder Chlor

Z $C_{1-12}$-Alkyl, $COR^5$ oder $CONHR^5$, wo $R^5$ für aromatische oder aliphatische Reste steht

$R^1$ Wasserstoff, $C_{1-12}$-Alkyl oder Chlor.

2. 3-Chlor-2-hydroxy-caprophenon-oxim
5-Chlor-2-hydroxy-caprophenon-oxim
5-Methyl-2-hydroxy-caprophenon-oxim
5-Methyl-2-hydroxy-caprophenon- (N-phenyl-carbamoyl)-oxim
3-Chlor-2-hydroxy-laurophenon-oxim
5-Chlor-2-hydroxy-laurophenon-oxim
4-n-Butoxy-2-hydroxy-acetophenon-oxim
4-n-Pentoxy-2-hydroxy-acetophenon-oxim
4-n-Decyloxy-2-hydroxy-acetophenon-oxim
4-n-Octadecyloxy-2-hydroxy-acetophenon-oxim
4-n-Butoxy-5-n-hexyl-2-hydroxy-acetophenon-oxim
4-Benzyloxy-2-hydroxy-acetophenon-oxim
4-n-Decyloxy-2-hydroxy-propiophenon-oxim
4-n-Dodecyloxy-2-hydroxy-propiophenon-oxim
4-Cyclohexyloxy-2-hydroxy-propiophenon-oxim
4-n-Pentoxy-2-hydroxy-caprophenon-oxim
4-n-Decyloxy-2-hydroxy-caprophenon-oxim
4-n-Octyloxy-2-hydroxy-laurophenon-oxim
4-n-Decyloxy-2-hydroxy-laurophenon-oxim

3. Verwendung von pharmazeutischen Zusammensetzungen nach Anspruch 1 oder von Verbindungen nach Anspruch 2 zur Herstellung eines Arzneimittels zum Einsatz als universeller Lipoxygenasehemmer.

4. Mittel zur Behandlung aller Formen des Asthma bronchiale, der asthmoiden Bronchitis und des obstruktiven Lungenemphysems sowie aller übrigen broncho-konstriktorischen Zustände, gekennzeichnet durch den Gehalt eines oder mehrerer Wirkstoffe gemäß Anspruch 1 oder 2.

5. Mittel zur Behandlung aller allergischen Erkrankungen, insbesondere atopische Dermatitis, allergische Rhinitis, Urticaria, Angiooedem, Kontaktdermatitis, allergische Konjunktivitis und allergische Erkrankungen des Gastrointestinaltraktes, gekennzeichnet durch den Gehalt eines oder mehrerer Wirkstoffe gemäß Anspruch 1 oder 2.

6. Mittel zur Behandlung von entzündlichen Erkrankungen, insbesondere solchen, bei denen die herkömmlichen Antiphlogistika, deren Angriffspunkt nicht die Lipoxygenase ist, ungenügende therapeutische Effekte zeigen, insbesondere bei purulenten Entzündungen sowie bei rheumatischen und arthritischen Erkrankungen, gekennzeichnet durch den Gehalt eines oder mehrerer Wirkstoffe gemäß Anspruch 1 oder 2.

7. Mittel zur Behandlung aller Formen der Thrombose, insbesondere der Thrombophlebitis, und bei Thromboseprophylaxe bei chronisch-ischämischer Herzkrankheit, Nachbehandlung bei Myokardinfekt, chronisch-rezidivierender Thrombose und chronischer Thrombophlebitis, gekennzeichnet durch den Gehalt eines oder mehrerer Wirkstoffe gemäß Anspruch 1 oder 2.

8. Mittel zur Verwendung als antiarteriosklerotische, gastroprotektive oder antimetastatische Arzneimittel, gekennzeichnet durch den Gehalt eines oder mehrerer Wirkstoffe gemäß Anspruch 1 oder 2.

9. Mittel zur Behandlung aller Formen des arteriellen Hochdrucks, insbesondere auch des arteriellen Hochdrucks (im Lungenkreislauf), gekennzeichnet durch den Gehalt eines oder mehrerer Wirkstoffe gemäß Anspruch 1 oder 2.

10. Mittel zur Verwendung als Spasmolytika zur Behandlung spastischer Zustände der glatten Muskulatur verschiedener Genese, insbesondere in verschiedenen Abschnitten des Verdauungs- und

Urogenitaltraktes sowie der Blutgefäßmuskulatur, gekennzeichnet durch den Gehalt eines oder mehrerer Wirkstoffe gemäß Anspruch 1 oder 2.

11. Arzneimittel, enthaltend mindestens eine Verbindung der Formel I gemäß Anspruch 1 in Kombination mit mindestens einem der traditionellen antiallergischen, antiasthmatischen, antiphlogistischen, antihypertensiven, spasmolytischen, antiarteriosklerotischen, antimetastatischen und antithrombotischen Wirkstoffe.

12. Verfahren zur Herstellung von Arzneimitteln, dadurch gekennzeichnet, daß pharmazeutische Zusammensetzungen nach Anspruch 1 oder Verbindungen nach Anspruch 2, gegebenenfalls zusammen mit Hilfs- und Trägerstoffen, in eine geeignete Applikationsform überführt werden.

**Patentansprüche für den Vertragsstaat: AT**

1. Pharmazeutische Zusammensetzungen, gekennzeichnet durch einen Gehalt an 1-(2-Hydroxyaryl)-alkan-1-on-oximen der allgemeinen Formel (I)

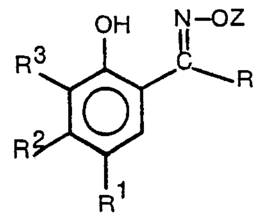

I

als Wirkstoff, worin bedeuten:

a) R $C_{11-18}$-Alkyl für $R^2$, $R^3$ und Z gleich Wasserstoff $R^1$ Wasserstoff, $C_{1-12}$-Alkyl oder Chlor oder

b) R $C_{1-18}$-Alkyl oder Aralkyl für $R^2$ $C_{1-12}$-Alkyl oder $OR^4$, wo $R^4$ für $C_{1-18}$-Alkyl, $C_{3-8}$-Cycloalkyl oder Aralkyl steht

$R^3$ $C_{1-12}$-Alkyl oder Chlor

Z $C_{1-12}$-Alkyl, $COR^5$ oder $CONHR^5$, wo $R^5$ für aromatische oder aliphatische Reste steht

$R^1$ Wasserstoff, $C_{1-12}$-Alkyl oder Chlor.

2. 3-Chlor-2-hydroxy-caprophenon-oxim
5-Chlor-2-hydroxy-caprophenon-oxim
5-Methyl-2-hydroxy-caprophenon-oxim
5-Methyl-2-hydroxy-caprophenon- (N-phenyl-carbamoyl)-oxim
3-Chlor-2-hydroxy-laurophenon-oxim
5-Chlor-2-hydroxy-laurophenon-oxim
4-n-Butoxy-2-hydroxy-acetophenon-oxim
4-n-Pentoxy-2-hydroxy-acetophenon-oxim
4-n-Decyloxy-2-hydroxy-acetophenon-oxim
4-n-Octadecyloxy-2-hydroxy-acetophenon-oxim
4-n-Butoxy-5-n-hexyl-2-hydroxy-acetophenon-oxim
4-Benzyloxy-2-hydroxy-acetophenon-oxim
4-n-Decyloxy-2-hydroxy-proiophenon-oxim
4-n-Dodecyloxy-2-hydroxy-propiophenon-oxim
4-Cyclohexyloxy-2-hydroxy-propiophenon-oxim
4-n-Pentoxy-2-hydroxy-caprophenon-oxim
4-Decyloxy-2-hydroxy-caprophenon-oxim
4-n-Octyloxy-2-hydroxy-laurophenon-oxim
4-n-Decyloxy-2-hydroxy-laurophenon-oxim

3. Verwendung von pharmazeutischen Zusammensetzungen nach Anspruch 1 oder von Verbindungen nach Anspruch 2 zur Herstellung eines Arzneimittels zum Einsatz als universeller Lipoxygenasehemmer.

4. Mittel zur Behandlung aller Formen des Asthma bronchiale, der asthmoiden Bronchitis und des obstruktiven Lungenemphysems sowie aller übrigen broncho-konstriktorischen Zustände, gekennzeichnet durch den Gehalt eines oder mehrerer Wirkstoffe gemäß Anspruch 1 oder 2.

5. Mittel zur Behandlung aller allergischen Erkrankungen, insbesondere atopische Dermatitis, allergische Rhinitis, Urticaria, Angiooedem, Kontaktdermatitis, allergische Konjunktivitis und allergische Erkrankungen des Gastrointestinaltraktes, gekennzeichnet durch den Gehalt eines oder mehrerer Wirkstoffe gemäß Anspruch 1 oder 2.

6. Mittel zur Behandlung von entzündlichen Erkrankungen, insbesondere solchen, bei denen die herkömmlichen Antiphlogistika, deren Angriffspunkt nich die Lipoxygenase ist, ungenügende therapeutische Effekte zeigen, insbesondere bei purulenten Entzündungen sowie bei rheumatischen und arthritischen Erkrankungen, gekennzeichnet durch den Gehalt eines oder mehrerer Wirkstoffe gemäß Anspruch 1 oder 2.

7. Mittel zur Behandlung aller Formen der Thrombose, insbesondere der Thrombophlebitis, und bei

Thromboseprophylaxe bei chronisch-ischämischer Herzkrankheit, Nachbehandlung bei Myokardinfekt, chronisch-rezidivierender Thrombose und chronischer Thrombophlebitis, gekennzeichnet durch den Gehalt eines oder mehrerer Wirkstoffe gemäß Anspruch 1 oder 2.

8. Mittel zur Verwendung als antiarteriosklerotische, gastroprotektive oder antimetastatische Arzneimittel, gekennzeichnet durch den Gehalt eines oder mehrerer Wirkstoffe gemäß Anspruch 1 oder 2.

9. Mittel zur Behandlung aller Formen des arteriellen Hochdrucks, insbesondere auch des arteriellen Hochdrucks (im Lungenkreislauf), gekennzeichnet durch den Gehalt eines oder mehrerer Wirkstoffe gemäß Anspruch 1 oder 2.

10. Mittel zur Verwendung als Spasmolytika zur Behandlung spastischer Zustände der glatten Muskulatur verschiedener Genese, insbesondere in verschiedenen Abschnitten des Verdauungs- und Urogenitaltraktes sowie der Blutgefäßmuskulatur, gekennzeichnet durch den Gehalt eines oder mehrerer Wirkstoffe gemäß Anspruch 1 oder 2.

11. Arzneimittel, enthaltend mindestens eine Verbindung der Formel I gemäß Anspruch 1 in Kombination mit mindestens einem der traditionellen antiallergischen, antiasthmatischen, antiphlogistischen, antihypertensiven, spasmolytischen, antiarteriosklerotischen, antimetastatischen und antithrombotischen Wirkstoffe.

12. Verfahren zur Herstellung von Arzneimitteln, dadurch gekennzeichnet, daß pharmazeutische Zusammensetzungen nach Anspruch 1 oder Verbindungen nach Anspruch 2, gegebenenfalls zusammen mit Hilfs- und Trägerstoffen, in eine geeignete Applikationsform überführt werden.

13. Verfahren zur Herstellung von Verbindungen der allgemeinen Formel I, bei dem man eine aliphatische Carbonsäure $C_nH_{2n+1}COOH$ oder ein Derivat davon, vorzugsweise ein Carbonsäurechlorid unter Friedel-Crafts- oder Friedel-Crafts-ähnlichen Acylierungsbedingungen mit einem substituierten Phenol zu einem Keton umsetzt und das Keton unter Zusatz eines basischen Stoffs mit einem Hydroxylaminsalz zur Reaktion bringt, dadurch gekennzeichnet, daß man

a) als substituiertes Phenol p-Kresol und als Carbonsäure Capronsäure oder ein Derivat davon einsetzt, wobei man das nach der Umsetzung mit dem Hydroxylaminsalz erhaltene 5-Methyl-2-hydroxy-caprophenon-oxim wahlweise durch Addition von Phenylisocyanat weiter zum 5-Methyl-2-hydroxy-caprophenon-(N-phenyl-carbamoyl)-oxim umsetzt.

b) oder als substituiertes Phenol o-Chlorphenol oder p-Chlorphenol und als Carbonsäure Capronsäure oder Laurinsäure oder ein Derivat davon einsetzt.

14. Verfahren zur Herstellung von Verbindungen der allgemeinen Formel I, bei dem man ein 2,4-Dihydroxyphenon mit einem Alkylierungs- oder Arylierungsmittel in Gegenwart eines basischen Stoffs in 4-Stellung verethert und das veretherte Keton unter Zusatz eines basischen Stoffs gemäß Anspruch 13 zum Oxim umsetzt, dadurch gekennzeichnet, daß man,

a) als 2,4-Dihydroxyphenon 2,4-Dihydroxyacetophenon und als Alkylierungs- oder Arylierungsmittel n-Butylbromid, n-Pentylbromid, n-Decylbromid, n-Octylbromid, oder Benzylbromid oder jeweils das entsprechende Alkyl- oder Arylchlorid einsetzt,

b) als 2,4-Dihydroxyphenon 2,4-Dihydroxy-5-n-hexyl-acetophenon und als Alkylierungsmittel n-Butylbromid oder n-Butylchlorid einsetzt,

c) als 2,4-Dihydroxyphenon 2,4-Dihydroxypropiophenon und als Alkylierungsmittel n-Decylbromid, n-Dodecylbromid oder Cyclohexylbromid oder jeweils das entsprechende Alkylchlorid einsetzt,

d) als 2,4-Dihydroxyphenon 2,4-Dihydroxycaprophenon und als Alkylierungsmittel n-Pentylbromid oder n-Decylbromid oder jeweils das entsprechende Alkylchlorid einsetzt,

e) oder als 2,4-Dihydroxyphenon 2,4-Dihydroxylaurophenon und als Alkylierungsmittel n-Octylbromid oder n-Decylbromid oder jeweils das entsprechende Alkylchlorid einsetzt.

15. Verfahren nach Anspruch 14, dadurch gekennzeichnet, daß man die Veretherung in niedrig siedenden Alkylglykolen durchführt.

**Revendications pour les Etats contractants: BE CH DE FR GB IT LI NL SE**

1. Compostitions pharmaceutiques caractérisées en ce qu'elles contiennent des 1-(2-hydroxyaryl)-alcan-1-one-oximes de formule générale (I)

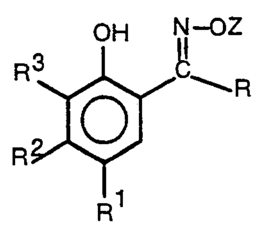

I

comme substances actives, où

a) R représente un alcoyle en $C_{11}$ à $C_{18}$ pour $R^2$, $R^3$ et Z = hydrogène $R^1$ représente un hydrogène, un alcoyle en $C_1$ à $C_{12}$ ou un chlore ou.

b) R représente un alcoyle en $C_1$ à $C_{18}$ ou un aralcoyle pour $R^2$ = alcoyle en $C_1$ à $C_{12}$ ou $OR^4$, où $R^4$ représente un alcoyle en $C_1$ à $C_{18}$, cycloalcoyle en $C_3$ à $C_8$ ou aralcoyle

$R^3$ représente un alcoyle en $C_1$ à $C_{12}$ ou un chlore

Z représente un alcoyle en $C_1$ à $C_{12}$, $COR^5$ ou $CONHR^5$, où $R^5$ représente des radicaux aromatiques ou aliphatiques

$R^1$ représente un hydrogène, alcoyle en $C_1$ à $C_{12}$ ou un chlore.

2. 3-chloro-2-hydroxy-caprophénon-oxime
5-chloro-2-hydroxy-caprophénon-oxime
5-méthyl-2-hydroxy-caprophénon-oxime
5-méthyl-2-hydroxy-caprophénon-(N-phényl-carbamoyl)-oxime
3-chloro-2-hydroxy-laurophénon-oxime
5-chloro-2-hydroxy-laurophénon-oxime
4-n-butoxy-2-hydroxy-acétophénon-oxime
4-n-pentoxy-2-hydroxy-acétophénon-oxime
4-n-décyloxy-2-hydroxy-acétophénon-oxime
4-n-octadécyloxy-2-hydroxy-acétophénon-oxime
4-n-butoxy-5-n-hexyl-2-hydroxy-acétophénon-oxime
4-benzyloxy-2-hydroxy-acétophénon-oxime
4-n-décyloxy-2-hydroxy-propiophénon-oxime
4-n-dodécyloxy-2-hydroxy-propiophénon-oxime
4-cyclohexyloxy-2-hydroxy-propiophénon-oxime
4-n-pentoxy-2-hydroxy-caprophénon-oxime
4-n-décyloxy-2-hydroxy-caprophénon-oxime
4-n-octyloxy-2-hydroxy-laurophénon-oxime
4-n-décyloxy-2-hydroxy-laurophénon-oxime.

3. Application de compositions pharmaceutiques selon la revendication 1 ou de composés selon la revendication 2 à la préparation d'un médicament aux fins d'utilisation comme inhibiteur universel de la lipoxygénase.

4. Agent de traitement de toutes les formes de l'asthme bronchique, de la bronchite asthmoïde et de l'emphysème pulmonaire obstructif ainsi que de tous les autres états de broncho-constriction, caractérisé en ce qu'il contient une ou plusieurs substances actives selon la revendication 1 ou 2.

5. Agent de traitement de toutes les maladies allergiques, en particulier la dermatite atopique, la rhinite allergique, l'urticaire, l'angio-oedème, la dermatite de contact, la conjonctivite allergique et les maladies allergiques de l'appareil gastrointestinal, caractérisé en ce qu'il contient une ou plusieurs substances actives selon la revendication 1 ou 2.

6. Agent de traitement des maladies inflammatoires, en particulier de celles dans lesquelles les anti-inflammatoires habituels, dont le point d'attaque n'est pas la lipoxygénase, présentent des effets thérapeutiques insuffisants, en particulier dans les inflammations purulentes ainsi que dans les maladies rhumatismales et arthritiques, caractérisé en ce qu'il contient une ou plusieurs substances actives selon la revendication 1 ou 2.

7. Agent de traitement de toutes les formes de la thrombose, en particulier de la thrombophlébite, et dans la prophylaxie de la thrombose en cas de maladie cardiaque ischémique chronique, le post-traitement dans l'infarctus de myocarde, la thrombose chronique récidivante et la thrombophlébite chronique, caractérisé en ce qu'il contient une ou plusieurs substances actives selon la revendication 1 ou 2.

8. Agent aux fins d'application comme médicament anti-artériosclérotique, gastro-protecteur ou anti-métastatique, caractérise en ce qu'il contient une ou plusieurs substances actives selon la revendication 1 ou 2.

9. Agent de traitement de toutes les formes de l'hypertension artérielle, en particulier également de l'hypertension artérielle dans la circulation pulmonaire, caractérisé en ce qu'il contient un ou plusieurs substances actives selon la revendication 1 ou 2.

10. Agent aux fins d'utilisation comme spasmolytique dans le traitement des états spasmodiques de la musculature lisse de différentes étiologies, en particulier dans différentes sections de l'appareil digestif et urogénital ainsi que de la musculature des vaisseaux sanguins, caractérisé en ce qu'il contient une ou plusieurs substances actives selon la revendication 1 ou 2.

11. Médicament contenant au moins un composé de formule I selon la revendication 1 en combinaison avec au moins une des matières actives anti-allergiques, anti-asthmatiques, anti-inflammatoires, anti-hypertensives, spasmolytiques, anti-artériosclérotiques, anti-métastatiques et anti-thrombotiques habituelles.

12. Procédé de préparation de médicaments, caractérisé en ce qu'on transforme des compositions pharmaceutiques selon la revendication 1 ou des composés selon la revendication 2, éventuellement avec des additifs et des supports, en une forme d'application appropriée.

**Revendications pour l'Etat contractant: AT**

1. Compositions pharmaceutiques caractérisées en ce qu'elles contiennent des 1-(2-hydroxyaryl)-alcan-1-one-oximdes de formule générale (I)

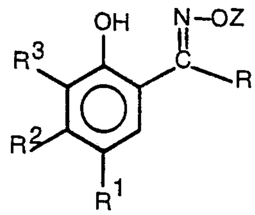

I

comme substances actives, où

a) R représente un alcoyle en $C_{11}$ à $C_{18}$ pour $R^2$, $R^3$ et Z = hydrogène $R^1$ représente un hydrogène, un alcoyle en $C_1$ à $C_{12}$ ou un chlore ou

b) R représente un alcoyle en $C_1$ à $C_{18}$ ou un aralcoyle pour $R^2$ = alcoyle en $C_1$ à $C_{12}$ ou $OR^4$, où $R^4$ représente un alcoyle en $C_1$ à $C_{18}$, cycloalcoyle en $C_3$ à $C_8$ ou aralcoyle

$R^3$ représente un alcoyle en $C_1$ à $C_{12}$ ou un chlore

Z représente un alcoyle en $C_1$ à $C_{12}$, $COR^5$ ou $CONHR^5$, où $R^5$ représente des radicaux aromatiques ou aliphatiques

$R^1$ représente un hydrogène, alcoyle en $C_1$ à $C_{12}$ ou un chlore.

2. 3-chloro-2-hydroxy-caprophénon-oxime
5-chloro-2-hydroxy-caprophénon-oxime
5-méthyl-2-hydroxy-caprophénon-oxime
5-méthyl-2-hydroxy-caprophénon-(N-phényl-carbamoyl)-oxime
3-chloro-2-hydroxy-laurophénon-oxime
4-n-butoxy-2-hydroxy-acétophénon-oxime
4-n-pentoxy-2-hydroxy-acétophénon-oxime
4-n-décyloxy-2-hydroxy-acétophénon-oxime
4-n-octadécyloxy-2-hydroxy-acétophénon-oxime
4-n-butoxy-5-n-hexyl-2-hydroxy-acétophénon-oxime
4-benzyoxy-2-hydroxy-acétophénon-oxime
4-n-décyloxy-2-hydroxy-propiophénon-oxime
4-n-dodécyloxy-2-hydroxy-propiophénon-oxime
4-cyclohexyloxy-2-hydroxy-propiophénon-oxime
4-n-pentoxy-2-hydroxy-caprophénon-oxime
4-n-décyloxy-2-hydroxy-caprophénon-oxime
4-n-octyloxy-2-hydroxy-laurophénon-oxime
4-n-décyloxy-2-hydroxy-laurophénon-oxime.

3. Application de compositions pharmaceutiques selon la revendication 1 ou de composés selon la revendication 2 à la préparation d'un médicament aux fins d'utilisation comme inhibiteur universel de lîpoxygénase.

4. Agent de traitement de toutes les formes de l'asthme bronchique, de la bronchite asthmoïde et de l'emphysème pulmonaire obstructif ainsi que de tous les autres états de broncho-constrictifs, caractérisé en ce qu'il contient une ou plusieurs substances actives selon la revendication 1 ou 2.

5. Agent de traitement de toutes les maladies allergiques, en particulier la dermatite atopique, de la rhinite allergique, l'urticaire, de l'angio-oedème, la dermatite de contact, de la conjonctivite allergique et des maladies allergiques de l'appareil gastro-intestinal, caractérisé en ce qu'il contient une ou plusieurs substances actives selon la revendication 1 ou 2.

6. Agent de traitement des maladies inflammatoires, en particulier de celles dans lesquelles les anti-inflammatoires habituels, dont le point d'attaque n'est pas la lipoxygénase, présentent des effets thérapeutiques insuffisants, en particulier dans les inflammations purulentes ainsi que dans les maladies rhumatismales et arthritiques, caractérisé en ce qu'il contient une ou plusieurs substances actives selon la revendication 1 ou 2.

7. Agent de traitement de toutes les formes de la thrombose, en particulier de la thrombophlébite, et de prophylaxie de la thrombose en cas de maladie cardiaque ischémique chronique, de post-traitement dans l'infarctus de myocarde, la thrombose chronique récidivante et la thrombophlébite chronique, caractérisé en ce qu'il contient une ou plusieurs substances actives selon la revendication 1 ou 2.

8. Agent aux fins d'application comme médicament antiartériosclérotique, gastro-protecteur ou anti-métastatique, caractérisé en ce qu'il contient une ou plusieurs substances actives selon la revendication 1 ou 2.

9. Agent de traitement de toutes les formes de l'hypertension artérielle, en particulier également de l'hypertension artérielle (dans la circulation pulmonaire), caractérisé en ce qu'il contient une ou plusieurs substances actives selon la revendication 1 ou 2.

10. Agent aux fins application comme spasmolytique dans le traitement des états spasmodiques de la musculature lisse de différentes étiologies, en particulier dans différentes sections de l'appareil digestif et uro-génital ainsi que de la musculature des vaisseaux sanguins, caractérisé en ce qu'il contient une ou plusieurs substances actives selon la revendication 1 ou 2.

11. Médicament contenant au moins un composé de formule I selon la revendication 1 en combinaison avec au moins une des substances actives anti-allergiques, anti-asthmatiques, anti-inflammatoires, anti-hypertensives, spasmolytiques, anti-artériosclérotiques, anti-métastatiques et anti-thrombotiques traditionnelles.

12. Procédé de préparation de médicaments, caractérisé en ce qu'on transforme des compositions pharmaceutiques selon la revendication 1 ou des composés selon la revendication 2, éventuellement avec des additifs et des supports, en une forme d'application appropriée.

13. Procédé de préparation de composés de formule générale I, dans lequel on fait réagir un acide carboxylique aliphatique $C_nH_{2n+1}COOH$ ou un de ses dérivés, de préférence un chlorure d'acide carboxylique dans des conditions d'acylation de Friedel-Crafts ou analogues à Friedel-Crafts avec un phénol substitué pour donner une cétone et en ce qu'on fait réagir la cétone avec addition d'une substance basique avec un sel d'hydroxylamine, caractérisé en ce que

a) on utilise comme phénol substitué le p-crésol et comme acide carboxylique l'acide caproïque ou un de ses dérivés, où l'on fait ensuite réagir si on le désire le 5-méthyl-2-hydroxy-caprophénon-oxime obtenu après la réaction avec le sel d'hydroxylamine par addition l'isocyanate de phényle pour donner le 5-méthyl-2-hydroxy-caprophénon-(N-phényl-carbamoyl)-oxime,

b) ou on utilise comme phénol substitué le o-chlorophénol ou le p-chlorophénol et comme acide carboxylique l'acide caproïque ou l'acide laurique ou un de ses dérivés.

14. Procédé de préparation de composés de formule générale I, dans lequel on éthérifie en position 4 une 2,4-dihydroxyphénone avec un agent d'alcoylation ou d'arylation en présence d'une substance basique et on fait réagir la cétone éthérifiée avec addition d'une substance basique selon la revendication 13 pour donner l'oxime, caractérisé en ce que

a) on utilise comme 2,4-dihydroxyphénone la 2,4-dihydroxyacétophénone et comme agent d'alcoylation ou d'arylation le bromure de n-butyle, le bromure de n-pentyle, le bromure de n-décyle, le bromure de n-octyle ou le bromure de benzyle ou à chaque fois le chlorure d'alcoyle ou d'aryle correspondant,

b) on utilise comme 2,4-dihydroxyphénone la 2,4-dihydroxy-5-n-hexyl-acétophénone et comme agent d'alcoylation le bromure de n-butyle ou le chlorure de n-butyle,

c) on utilise comme 2,4-dihydroxyphénone la 2,4-dihydroxypropiophénone et comme agent d'alcoylation le bromure de n-décyle, le bromure de n-dodécyle ou le bromure de cyclohexyle ou à chaque fois le chlorure d'alcoyle correspondant,

d) on utilise comme 2,4-dihydroxyphénone la 2,4-dihydroxycaprophénone et comme agent d'alcoylation le bromure de n-pentyle ou le bromure de n-décyle ou à chaque fois le chlorure d'alcoyle correspondant,

e) ou on utilise comme 2,4-dihydroxyphénone la 2,4-dihydroxylaurophénone et comme agent d'alcoylation le bromure de n-octyle ou le bromure de n-décyle ou à chaque fois le chlorure d'alcoyle correspondant.

15. Procédé selon la revendication 14, caractérisé en ce qu'on procède à l'éthérification dans des alcoylglycols à bas point d'ébullition.

**Claims for the Contracting States: BE CH DE FR GB IT LI NL SE**

1. Pharmaceutical compositions, characterized in that they contain 1-(2-hydroxyaryl)alkan-1-one oximes of general formula (I):

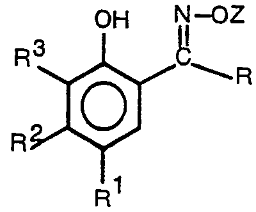

I

as the active ingredient, in which formula:

a) R is $C_{11-18}$ alkyl if $R^2$, $R^3$ and Z are hydrogen and $R^1$ is hydrogen, $C_{1-12}$ alkyl or chloro, or

b) R is $C_{1-18}$ alkyl or aralkyl if $R^2$ is $C_{1-12}$ or $OR^4$, where $R^4$ is $C_{1-18}$ alkyl, $C_{3-8}$ cycloalkyl or aralkyl, $R^3$ is $C_{1-12}$ alkyl or chloro, Z is $C_{1-12}$ alkyl, $COR^5$ or $CONHR^5$, where $R^5$ is an aromatic or aliphatic radical, and $R^1$ is hydrogen, $C_{1-12}$ alkyl or chloro.

2. 3-Chloro-2-hydroxycaprophenone oxime, 5-chloro-2-hydroxycaprophenone oxime, 5-methyl-2-hydroxycaprophenone oxime, 5-methyl-2-hydroxycaprophenone (N-phenylcarbamoyl)oxime, 3-chloro-2-hydroxylaurophenone oxime, 5-chloro-2-hydroxylaurophenone oxime, 4-n-butoxy-2-hydroxyacetophenone oxime, 4-n-pentoxy-2-hydroxyacetophenone oxime, 4-n-decyloxy-2-hydroxyacetophenone oxime, 4-n-octadecyloxy-2-hydroxyacetophenone oxime, 4-n-butoxy-5-n-hexyl-2-hydroxyacetophenone oxime, 4-benzyloxy-2-hydroxyacetophenone oxime, 4-n-decyloxy-2-hydroxypropiophenone oxime, 4-n-dodecyloxy-2-hydroxypropiophenone oxime, 4-cyclohexyloxy-2-hydroxypropiophenone oxime, 4-n-pentoxy-2-hydroxycaprophenone oxime, 4-n-decyloxy-2-hydroxycaprophenone oxime, 4-n-octyloxy-2-hydroxylaurophenone oxime and 4-n-decyloxy-2-hydroxylaurophenone oxime.

3. Use of pharmaceutical compositions according to Claim 1 or of compounds according to Claim 2 for the preparation of a drug to be used as a universal lipoxygenase inhibitor.

4. Compositions for the treatment of all forms of bronchial asthma, asthmoid bronchitis and obstructive pulmonary emphysema, as well as all other bronchoconstrictory conditions, characterized in that they contain one or more active ingredients according to Claim 1 or Claim 2.

5. Compositions for the treatment of all allergic diseases, especially atopic dermatitis, allergic rhinitis, urticaria, angioneurotic oedema, contact dermatitis, allergic conjunctivitis and allergic diseases of the gastrointestinal tract, characterized in that they contain one or more active ingredients according to Claim 1 or Claim 2.

6. Compositions for the treatment of inflammatory diseases, especially those where the common antiphlogistics, whose site of action is other than lipoxygenase, exhibit inadequate therapeutic effects, especially for purulent inflammations and rheumatic and arthritic diseases, characterized in that they contain one or more active ingredients according to Claim 1 or Claim 2.

7. Compositions for the treatment of all forms of thrombosis, especially thrombophlebitis, and for the prophylaxis of thrombosis in chronically ischaemic heart disease, follow-up treatment after myocardial infarction, chronically recurrent thrombosis and chronic thrombophlebitis, characterized in that they contain one or more active ingredients according to Claim 1 or Claim 2.

8. Compositions for use as antiarteriosclerotic, gastroprotective or antimetastatic drugs, characterized in that they contain one or more active ingredients according to Claim 1 or Claim 2.

9. Compositions for the treatment of all forms of arterial hypertension, including especially pulmonary hypertension (in the pulmonary circulation), characterized in that they contain one or more active ingredients according to Claim 1 or Claim 2.

10. Compositions for use as spasmolytics for the treatment of spastic conditions of the smooth musculature of various origins, especially in different sections of the digestive and urogential tracts and the blood vessel musculature, characterized in that they contain one or more active ingredients according to Claim 1 or Claim 2.

11. Drugs containing at least one compound of formula I according to Claim 1, in combination with at least one of the traditional antiallergic, antiasthmatic, antiphlogistic, antihypertensive, splasmolytic, antiarteriosclerotic, antimetastatic and antithrombotic active ingredients.

12. A method of preparing drugs, characterized in that pharmaceutical compositions according to Claim 1 or compounds according to Claim 2, together with adjuncts and excipients if appropriate, are converted to a suitable form of administration.

**Claims for the Contracting State: AT**

1. Pharmaceutical compositions, characterized in that they contain 1-(2-hydroxyaryl)alkan-1-one oximes of general formula (I):

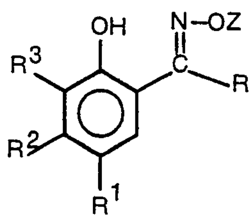

I

as the active ingredient, in which formula:

a) R is $C_{11-18}$ alkyl if $R^2$, $R^3$ and Z are hydrogen and $R^1$ is hydrogen $C_{1-12}$ alkyl or chloro, or

b) R is $C_{1-18}$ alkyl or aralkyl if $R^2$ is $C_{1-12}$ alkyl or $OR^4$, where $R^4$ is $C_{1-18}$ alkyl, $C_{3-8}$ cycloalkyl or aralkyl, $R^3$ is $C_{1-12}$ alkyl or chloro, Z is $C_{1-12}$ alkyl, $COR^5$ or $CONHR^5$, where $R^5$ is an aromatic or aliphatic radical, and $R^1$ is hydrogen, $C_{1-12}$ alkyl or chloro.

2. 3-Chloro-2-hydroxycaprophenone oxime, 5-chloro-2-hydroxycaprophenone oxime, 5-methyl-2-hydroxycaprophenone oxime, 5-methyl-2-hydroxycaprophenone (N-phenylcarbamoyl)oxime, 3-chloro-2-hydroxylaurophenone oxime, 5-chloro-2-hydroxylaurophenone oxime, 4-n-butoxy-2-hydroxyacetophenone oxime, 4-n-pentoxy-2-hydroxyacetophenone oxime, 4-n-decyloxy-2-hydroxyacetophenone oxime, 4-n-octadecyloxy-2-hydroxyacetophenone oxime, 4-n-butoxy-5-n-hexyl-2-hydroxyacetophenone oxime, 4-benzyloxy-2-hydroxyacetophenone oxime, 4-n-decyloxy-2-hydroxy-

propiophenone oxime, 4-n-dodecyloxy-2-hydroxypropiophenone oxime, 4-cyclohexyloxy-2-hydroxypropiophenone oxime, 4-n-pentoxy-2-hydroxycaprophenone oxime, 4-n-decyloxy-2-hydroxycaprophenone oxime, 4-n-octyloxy-2-hydroxylaurophenone oxime and 4-n-decyloxy-2-hydroxylaurophenone oxime.

3. Use of pharmaceutical compositions according to Claim 1 or of compounds according to Claim 2 for the preparation of a drug to be used as a universal lipoxygenase inhibitor.

4. Compositions for the treatment of all forms of bronchial asthma, asthmoid bronchitis and obstructive pulmonary emphysema, as well as all other bronchoconstrictory conditions, characterized in that they contain one or more active ingredients according to Claim 1 or Claim 2.

5. Compositions for the treatment of all allergic diseases, especially atopic dermatitis, allergic rhinitis, urticaria, angioneurotic oedema, contact dermatitis, allergic conjunctivitis and allergic dseases of the gastrointestinal tract, characterized in that they contain one or more active ingredients according to Claim 1 or Claim 2.

6. Compositions for the treatment of inflammatory diseases, especially those where the common antiphlogistics, whose site of action is other than lipoxygenase, exhibit inadequate therapeutic effects, especially for purulent inflammations and rheumatic and arthritic diseases, characterized in that they contain one or more active ingredients according to Claim 1 or Claim 2.

7. Compositions for the treatment of all forms of thrombosis, especially thrombophlebitis, and for the prophylaxis of thrombosis in chronically ischaemic heart disease, follow-up treatment after myocardial infarction, chronically recurrent thrombosis and chronic thrombophlebitis, characterized in that they contain one or more active ingredients according to Claim 1 or Claim 2.

8. Compositions for use as antiarteriosclerotic, gastroprotective or antimetastatic drugs, characterized in that they contain one or more active ingredients according to Claim 1 or Claim 2.

9. Compositions for the treatment of all forms of arterial hypertension, including especially pulmonary hypertension (in the pulmonary circulation), characterized in that they contain one or more active ingredients according to Claim 1 or Claim 2.

10. Compositions for use as spasmolytics for the treatment of spastic conditions of the smooth musculature of various origins, especially in different sections of the digestive and urogenital tracts and the blood vessel musculature, characterized in that they contain one or more active ingredients according to Claim 1 or Claim 2.

11. Drugs containing at least one compound of formula I according to Claim 1, in combination with at least one of the traditional antiallergic, antiasthmatic, antiphlogistic, antihypertensive, splasmolytic, antiarteriosclerotic, antimetastatic and antithrombotic active ingredients.

12. A method of preparing drugs, characterized in that pharmaceutical compositions according to Claim 1 or compounds according to Claim 2, together with adjuncts and excipients if appropriate, are converted to a suitable form of administration.

13. A method for making compounds of general formula (I) comprising reacting an aliphatic carboxylic acid $C_nH_{2n-1}COOH$ or a derivative thereof, preferably a carboxylic chloride under Friedel Crafts acylating conditions or acylating conditions similar thereto, with a substituted phenol to a ketone, and reacting said ketone with a hydroxylamine salt, wherein a basic compound is added, characterized by

a) using p-cresol as said substituted phenol, and capronic acid or a derivative thereof as said carboxylic acid, wherein the 5-methyl-2-hydroxy-caprophenone-oxime obtained after reaction with said hydroxylamine salt is optionally further reacted to 5-methyl-2-hydroxy-caprophenone-(N-phenyl-carbamoyl)-oxime by addition of phenylisocyanate,

b) or using o-chlorophenol or p-chlorophenol as said substituted phenol, and capronic acid or lauric acid or a derivative thereof as said carboxylic acid.

14. A method for making compounds of general formula (I) comprising etherifying a 2,4-dihydroxyphenone in the 4-position with an alkylating or arylating agent in the presence of a basic compound, and reacting said etherified ketone to said oxime in the presence of a basic compound in accordance with claim 13, characterized by

a) using 2,4-dihydroxyacetophenone as said 2,4-dihydroxyphenone, and n-butybromide, n-pentylbromide, n-decylbromide, n-octylbromide or benzylbromide or the corresponding alkyl chloride or aryl chloride as said alkylating agent or arylating agent,

b) using 2,4-dihydroxy-5-n-hexyl-acetophenone as said 2,4-dihydroxyphenone, and n-butylbromide or n-butylchloride as said alkylating agent,

c) using 2,4-dihydroxypropiophenone as said 2,4-dihydroxyphenone, and n-decylbromide, n-dodecylbromide or cyclohexylbromide or the corresponding alkylchloride as said alkylating agent,

d) using 2,4-dihydroxycaprophenone as said 2,4-dihydroxyphenone, and n-pentylbromide or n-decylbromide or the corresponding alcylchloride as said alkylating agent,

e) or using 2,4-dihydroxylaurophenone as said 2,4-dihydroxyphenone, and n-octylbromide or n-decylbromide or the corresponding alkylchloride as said alkylating agent.

15. The method of claim 14, characterized in that etheryfication is carried out in low boiling alkylglycoles.

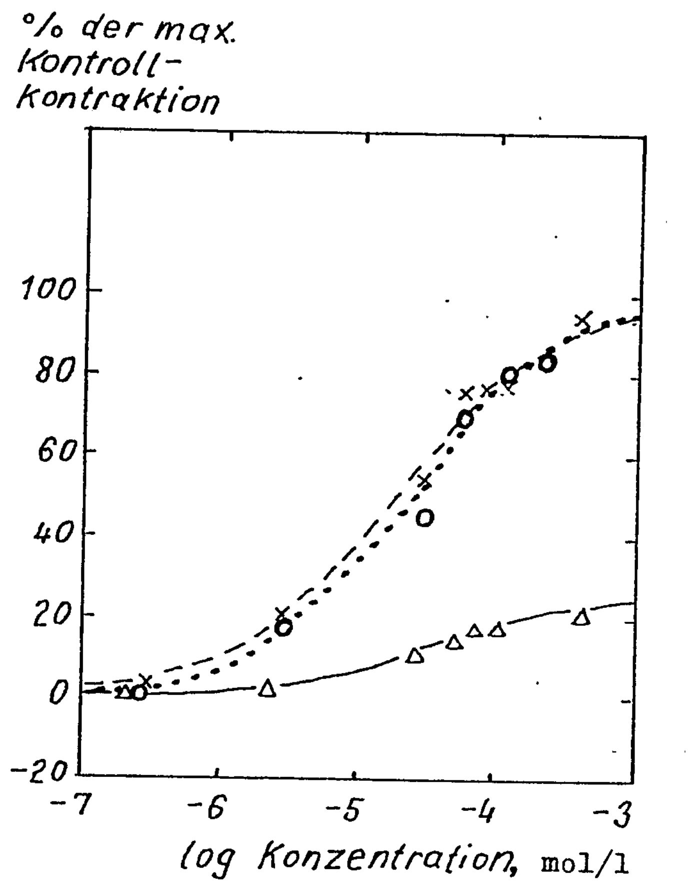


x : Lösungsmittel (Blindversuch)
o : R = Methyl

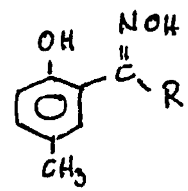

Δ : R = $C_{11}$